# EUROPEAN PATENT APPLICATION

(11) **EP 3 882 257 A1**
(43) Date of publication of application: **22.09.2021**
(21) Application number: 20164689.0
(22) Date of filing: 20.03.2020
(51) Int. Cl.: C07K 7/06, C07K 14/52, C12N 9/48

(54) **NGR CONJUGATES AND USES THEREOF**

(71) Applicant: Ospedale San Raffaele S.r.l., 20132 Milano (IT)
(72) Inventor: CURNIS, Flavio, 20132 Milano (IT); CORTI, Angelo, 20132 Milano (IT); FERRERI, Andrès J.M., 20132 Milano (IT)
(74) Representative: Turri, Elisa

(57) **Abstract**

The present invention relates to a conjugate comprising a first peptide of sequence CNGRCG (SEQ ID NO:1) linked to the N-terminus of a protein and a compound X linked to the N-terminus of said peptide and to related medical uses.

SEQUENCE LISTING

<110>  Ospedale San Raffaele S.r.l.

<120>  NGR CONJUGATES AND USES THEREOF

<130>  BE 141643

<160>  43

<170>  PatentIn version 3.5

<210>  1
<211>  6
<212>  PRT
<213>  Homo sapiens

<400>  1

Cys Asn Gly Arg Cys Gly
1               5

<210>  2
<211>  4
<212>  PRT
<213>  Homo sapiens

<400>  2

Ile Glu Gly Arg
1

<210>  3
<211>  10
<212>  PRT
<213>  Homo sapiens

<400>  3

Ser Cys Asn Gly Arg Cys Gly Val Arg Tyr
1               5                   10

<210>  4
<211>  9
<212>  PRT
<213>  Homo sapiens

<400>  4

Cys Asn Gly Arg Cys Gly Val Arg Tyr
1               5

<210>  5
<211>  5
<212>  PRT
<213>  Homo sapiens

<400>  5

Cys Asn Gly Arg Cys
1               5

<210>  6
<211>  7
<212>  PRT
<213>  Homo sapiens

<400>  6

Ser Cys Asn Gly Arg Cys Gly
1               5

<210>  7
<211>  5
<212>  PRT
<213>  Homo sapiens

<400>  7

Lys Asn Gly Arg Glu
1               5

<210>  8
<211>  6
<212>  PRT
<213>  Homo sapiens

<400>  8

Cys Gly Asn Gly Arg Gly
1               5

<210>  9
<211>  5
<212>  PRT
<213>  Homo sapiens

<400>  9

Gly Asn Gly Arg Gly
1               5

<210>  10
<211>  6
<212>  PRT
<213>  Homo sapiens

<400>  10

Asn Gly Arg Ala His Ala
1               5

<210>  11
<211>  5
<212>  PRT
<213>  Homo sapiens

<400>  11

Cys Asp Gly Arg Cys
1               5


<210>  12
<211>  9
<212>  PRT
<213>  Homo sapiens

<400>  12

Cys Val Leu Asn Gly Arg Met Glu Cys
1               5


<210>  13
<211>  6
<212>  PRT
<213>  Homo sapiens

<400>  13

Asn Gly Arg Ala Gly Gly
1               5


<210>  14
<211>  157
<212>  PRT
<213>  Homo sapiens

<400>  14

<210>  15
<211>  156
<212>  PRT
<213>  Mus musculus

<400>  15

Leu Arg Ser Ser Ser Gln Asn Ser Ser Asp Lys Pro Val Ala His Val
1               5                   10                  15

Val Ala Asn His Gln Val Glu Glu Gln Leu Glu Trp Leu Ser Gln Arg
            20                  25                  30

Ala Asn Ala Leu Leu Ala Asn Gly Met Asp Leu Lys Asp Asn Gln Leu
        35                  40                  45

Val Val Pro Ala Asp Gly Leu Tyr Leu Val Tyr Ser Gln Val Leu Phe
    50                  55                  60

Lys Gly Gln Gly Cys Pro Asp Tyr Val Leu Leu Thr His Thr Val Ser
65                  70                  75                  80

Arg Phe Ala Ile Ser Tyr Gln Glu Lys Val Asn Leu Leu Ser Ala Val
                85                  90                  95

Lys Ser Pro Cys Pro Lys Asp Thr Pro Glu Gly Ala Glu Leu Lys Pro
            100                 105                 110

Trp Tyr Glu Pro Ile Tyr Leu Gly Gly Val Phe Gln Leu Glu Lys Gly
        115                 120                 125

Asp Gln Leu Ser Ala Glu Val Asn Leu Pro Lys Tyr Leu Asp Phe Ala
    130                 135                 140

Glu Ser Gly Gln Val Tyr Phe Gly Val Ile Ala Leu
145                 150                 155

<210>  16
<211>  20
<212>  PRT

<213>  Escherichia coli

<400>  16

Met Arg Ala Lys Leu Leu Gly Ile Val Leu Thr Thr Pro Ile Ala Ile
1               5                   10                  15

Ser Ser Phe Ala
            20

<210>  17
<211>  8
<212>  PRT
<213>  Homo sapiens

<220>
<221>  MOD_RES
<222>  (1)..(1)
<223>  acetyl-Ciso or Ciso or SCiso

<400>  17

Asp Gly Arg Cys Gly Val Arg Tyr
1               5

<210>  18
<211>  15
<212>  PRT
<213>  Homo sapiens

<400>  18

Cys Asn Gly Arg Cys Gly Val Arg Ser Ser Ser Arg Thr Pro Ser
1               5                   10                  15

<210>  19
<211>  11
<212>  PRT
<213>  Homo sapiens

<400>  19

Ala Cys Asp Cys Arg Gly Asp Cys Phe Cys Gly
1               5                   10

<210>  20
<211>  7
<212>  PRT
<213>  Homo sapiens

<400>  20

Ser Gly Cys Asn Gly Arg Cys
1               5

<210>  21

<211>  22
<212>  PRT
<213>  Homo sapiens

<400>  21

Leu Leu Gly Ile Val Leu Thr Thr Pro Ile Ala Ile Ser Ser Phe Ala
1               5                   10                  15

Ser Thr Cys Asn Gly Arg
            20

<210>  22
<211>  10
<212>  PRT
<213>  Homo sapiens

<400>  22

Ser Cys Asp Gly Arg Cys Gly Val Arg Tyr
1               5                   10

<210>  23
<211>  19
<212>  PRT
<213>  Saccharomyces cerevisiae

<400>  23

Met Arg Phe Pro Ser Ile Phe Thr Ala Val Leu Phe Ala Ala Ser Ser
1               5                   10                  15

Ala Leu Ala

<210>  24
<211>  184
<212>  PRT
<213>  Artificial Sequence

<220>
<223>  conjugate

<400>  24

Met Arg Ala Lys Leu Leu Gly Ile Val Leu Thr Thr Pro Ile Ala Ile
1               5                   10                  15

Ser Ser Phe Ala Ser Thr Cys Asn Gly Arg Cys Gly Leu Arg Ser Ser
            20                  25                  30

Ser Gln Asn Ser Ser Asp Lys Pro Val Ala His Val Val Ala Asn His
        35                  40                  45

Gln Val Glu Glu Gln Leu Glu Trp Leu Ser Gln Arg Ala Asn Ala Leu

50                  55                  60

Leu Ala Asn Gly Met Asp Leu Lys Asp Asn Gln Leu Val Val Pro Ala
65                  70                  75                  80

Asp Gly Leu Tyr Leu Val Tyr Ser Gln Val Leu Phe Lys Gly Gln Gly
                85                  90                  95

Cys Pro Asp Tyr Val Leu Leu Thr His Thr Val Ser Arg Phe Ala Ile
            100                 105                 110

Ser Tyr Gln Glu Lys Val Asn Leu Leu Ser Ala Val Lys Ser Pro Cys
        115                 120                 125

Pro Lys Asp Thr Pro Glu Gly Ala Glu Leu Lys Pro Trp Tyr Glu Pro
    130                 135                 140

Ile Tyr Leu Gly Gly Val Phe Gln Leu Glu Lys Gly Asp Gln Leu Ser
145                 150                 155                 160

Ala Glu Val Asn Leu Pro Lys Tyr Leu Asp Phe Ala Glu Ser Gly Gln
                165                 170                 175

Val Tyr Phe Gly Val Ile Ala Leu
            180

<210>  25
<211>  164
<212>  PRT
<213>  Artificial Sequence

<220>
<223>  conjugate

<400>  25

Met Ser Cys Asn Gly Arg Cys Gly Leu Arg Ser Ser Ser Gln Asn Ser
1               5                   10                  15

Ser Asp Lys Pro Val Ala His Val Val Ala Asn His Gln Val Glu Glu
            20                  25                  30

Gln Leu Glu Trp Leu Ser Gln Arg Ala Asn Ala Leu Leu Ala Asn Gly
        35                  40                  45

Met Asp Leu Lys Asp Asn Gln Leu Val Val Pro Ala Asp Gly Leu Tyr
    50                  55                  60

Leu Val Tyr Ser Gln Val Leu Phe Lys Gly Gln Gly Cys Pro Asp Tyr
65                  70                  75                  80

Val Leu Leu Thr His Thr Val Ser Arg Phe Ala Ile Ser Tyr Gln Glu
                85                  90                  95

Lys Val Asn Leu Leu Ser Ala Val Lys Ser Pro Cys Pro Lys Asp Thr
            100                 105                 110

Pro Glu Gly Ala Glu Leu Lys Pro Trp Tyr Glu Pro Ile Tyr Leu Gly
        115                 120                 125

Gly Val Phe Gln Leu Glu Lys Gly Asp Gln Leu Ser Ala Glu Val Asn
    130                 135                 140

Leu Pro Lys Tyr Leu Asp Phe Ala Glu Ser Gly Gln Val Tyr Phe Gly
145                 150                 155                 160

Val Ile Ala Leu

<210>  26
<211>  163
<212>  PRT
<213>  Artificial Sequence

<220>
<223>  conjugate

<400>  26

Ser Cys Asn Gly Arg Cys Gly Leu Arg Ser Ser Ser Gln Asn Ser Ser
1               5                   10                  15

Asp Lys Pro Val Ala His Val Val Ala Asn His Gln Val Glu Glu Gln
            20                  25                  30

Leu Glu Trp Leu Ser Gln Arg Ala Asn Ala Leu Leu Ala Asn Gly Met
        35                  40                  45

Asp Leu Lys Asp Asn Gln Leu Val Val Pro Ala Asp Gly Leu Tyr Leu
    50                  55                  60

Val Tyr Ser Gln Val Leu Phe Lys Gly Gln Gly Cys Pro Asp Tyr Val
65                  70                  75                  80

Leu Leu Thr His Thr Val Ser Arg Phe Ala Ile Ser Tyr Gln Glu Lys
                85                  90                  95

Val Asn Leu Leu Ser Ala Val Lys Ser Pro Cys Pro Lys Asp Thr Pro
            100                 105                 110

Glu Gly Ala Glu Leu Lys Pro Trp Tyr Glu Pro Ile Tyr Leu Gly Gly
        115                 120                 125

Val Phe Gln Leu Glu Lys Gly Asp Gln Leu Ser Ala Glu Val Asn Leu
    130                 135                 140

Pro Lys Tyr Leu Asp Phe Ala Glu Ser Gly Gln Val Tyr Phe Gly Val
145                 150                 155                 160

Ile Ala Leu

<210>  27
<211>  165
<212>  PRT
<213>  Artificial Sequence

<220>
<223>  conjugate

<400>  27

Met Ser Cys Asn Gly Arg Cys Gly Val Arg Ser Ser Ser Arg Thr Pro
1               5                   10                  15

Ser Asp Lys Pro Val Ala His Val Val Ala Asn Pro Gln Ala Glu Gly
            20                  25                  30

Gln Leu Gln Trp Leu Asn Arg Arg Ala Asn Ala Leu Leu Ala Asn Gly
        35                  40                  45

Val Glu Leu Arg Asp Asn Gln Leu Val Val Pro Ser Glu Gly Leu Tyr
    50                  55                  60

Leu Ile Tyr Ser Gln Val Leu Phe Lys Gly Gln Gly Cys Pro Ser Thr
65                  70                  75                  80

His Val Leu Leu Thr His Thr Ile Ser Arg Ile Ala Val Ser Tyr Gln
                85                  90                  95

Thr Lys Val Asn Leu Leu Ser Ala Ile Lys Ser Pro Cys Gln Arg Glu
            100                 105                 110

Thr Pro Glu Gly Ala Glu Ala Lys Pro Trp Tyr Glu Pro Ile Tyr Leu
        115                 120                 125

Gly Gly Val Phe Gln Leu Glu Lys Gly Asp Arg Leu Ser Ala Glu Ile
    130                 135                 140

Asn Arg Pro Asp Tyr Leu Asp Phe Ala Glu Ser Gly Gln Val Tyr Phe
145                 150                 155                 160

Gly Ile Ile Ala Leu
                165

<210>  28
<211>  164
<212>  PRT
<213>  Artificial Sequence

<220>
<223>  conjugate

<400>  28

Ser Cys Asn Gly Arg Cys Gly Val Arg Ser Ser Ser Arg Thr Pro Ser
1               5                   10                  15

Asp Lys Pro Val Ala His Val Val Ala Asn Pro Gln Ala Glu Gly Gln
            20                  25                  30

Leu Gln Trp Leu Asn Arg Arg Ala Asn Ala Leu Leu Ala Asn Gly Val
        35                  40                  45

Glu Leu Arg Asp Asn Gln Leu Val Val Pro Ser Glu Gly Leu Tyr Leu
    50                  55                  60

Ile Tyr Ser Gln Val Leu Phe Lys Gly Gln Gly Cys Pro Ser Thr His
65                  70                  75                  80

Val Leu Leu Thr His Thr Ile Ser Arg Ile Ala Val Ser Tyr Gln Thr
                85                  90                  95

Lys Val Asn Leu Leu Ser Ala Ile Lys Ser Pro Cys Gln Arg Glu Thr
            100                 105                 110

Pro Glu Gly Ala Glu Ala Lys Pro Trp Tyr Glu Pro Ile Tyr Leu Gly
        115                 120                 125

Gly Val Phe Gln Leu Glu Lys Gly Asp Arg Leu Ser Ala Glu Ile Asn
    130                 135                 140

Arg Pro Asp Tyr Leu Asp Phe Ala Glu Ser Gly Gln Val Tyr Phe Gly
145                 150                 155                 160

Ile Ile Ala Leu

<210>  29

<211>  182
<212>  PRT
<213>  Artificial Sequence

<220>
<223>  conjugate

<400>  29

Met Arg Phe Pro Ser Ile Phe Thr Ala Val Leu Phe Ala Ala Ser Ser
1               5                   10                  15

Ala Leu Ala Cys Asn Gly Arg Cys Gly Val Arg Ser Ser Ser Arg Thr
            20                  25                  30

Pro Ser Asp Lys Pro Val Ala His Val Val Ala Asn Pro Gln Ala Glu
        35                  40                  45

Gly Gln Leu Gln Trp Leu Asn Arg Arg Ala Asn Ala Leu Leu Ala Asn
    50                  55                  60

Gly Val Glu Leu Arg Asp Asn Gln Leu Val Val Pro Ser Glu Gly Leu
65                  70                  75                  80

Tyr Leu Ile Tyr Ser Gln Val Leu Phe Lys Gly Gln Gly Cys Pro Ser
                85                  90                  95

Thr His Val Leu Leu Thr His Thr Ile Ser Arg Ile Ala Val Ser Tyr
            100                 105                 110

Gln Thr Lys Val Asn Leu Leu Ser Ala Ile Lys Ser Pro Cys Gln Arg
        115                 120                 125

Glu Thr Pro Glu Gly Ala Glu Ala Lys Pro Trp Tyr Glu Pro Ile Tyr
    130                 135                 140

Leu Gly Gly Val Phe Gln Leu Glu Lys Gly Asp Arg Leu Ser Ala Glu
145                 150                 155                 160

Ile Asn Arg Pro Asp Tyr Leu Asp Phe Ala Glu Ser Gly Gln Val Tyr
                165                 170                 175

Phe Gly Ile Ile Ala Leu
            180


<210>  30
<211>  564
<212>  DNA
<213>  Artificial Sequence

<220>

<223>   conjugate

<400>   30
catatgcggg cgaaactcct aggaatagtc ctgacaaccc ctatcgcgat cagctctttt      60

gcgtcgacgt gcaacggccg ttgtggcctc agatcatctt ctcaaaattc gagtgacaag     120

cctgtagccc acgtcgtagc aaaccaccaa gtggaggagc agctggagtg gctgagccag     180

cgcgccaacg ccctcctggc caacggcatg gatctcaaag acaaccaact agtggtgcca     240

gccgatgggt tgtaccttgt ctactcccag gttctcttca agggacaagg ctgccccgac     300

tacgtgctcc tcacccacac cgtcagccga tttgctatct cataccagga gaaagtcaac     360

ctcctctctg ccgtcaagag cccctgcccc aaggacaccc ctgagggggc tgagctcaaa     420

ccctggtatg agcccatata cctgggagga gtcttccagc tggagaaggg ggaccaactc     480

agcgctgagg tcaatctgcc caagtactta gactttgcgg agtccgggca ggtctacttt     540

ggagtcattg ctctgtgagg atcc                                            564


<210>   31
<211>   505
<212>   DNA
<213>   Artificial Sequence

<220>
<223>   conjugate

<400>   31
caccatgagc tgcaacggcc gttgcggcct cagatcatct tctcaaaatt cgagtgacaa      60

gcctgtagcc cacgtcgtag caaaccacca agtggaggag cagctggagt ggctgagcca     120

gcgcgccaac gccctcctgg ccaacggcat ggatctcaaa gacaaccaac tagtggtgcc     180

agccgatggg ttgtaccttg tctactccca ggttctcttc aagggacaag gctgccccga     240

ctacgtgctc ctcacccaca ccgtcagccg atttgctatc tcataccagg agaaagtcaa     300

cctcctctct gccgtcaaga gcccctgccc caaggacacc cctgaggggg ctgagctcaa     360

accctggtat gagcccatat acctgggagg agtcttccag ctggagaagg gggaccaact     420

cagcgctgag gtcaatctgc ccaagtactt agactttgcg gagtccgggc aggtctactt     480

tggagtcatt gctctgtgag gatcc                                           505


<210>   32
<211>   508
<212>   DNA
<213>   Artificial Sequence

<220>
<223>   conjugate

<400>   32
caccatgagc tgcaacggcc gttgcggcgt cagatcatct tctcgaaccc cgagtgacaa      60

gcctgtagcc catgttgtag caaaccctca agctgagggg cagctccagt ggctgaaccg 120

ccgggccaat gccctcctgg ccaatggcgt ggagctgaga gataaccagc tggtggtgcc 180

atcagagggc ctgtacctca tctactccca ggtcctcttc aagggccaag gctgcccctc 240

cacccatgtg ctcctcaccc acaccatcag ccgcatcgcc gtctcctacc agaccaaggt 300

caacctcctc tctgccatca agagcccctg ccagagggag accccagagg gggctgaggc 360

caagccctgg tatgagccca tctatctggg aggggtcttc cagctggaga agggtgaccg 420

actcagcgct gagatcaatc ggcccgacta tctcgacttt gccgagtctg ggcaggtcta 480

ctttgggatc attgccctgt gaggatcc 508

<210> 33
<211> 507
<212> DNA
<213> Artificial Sequence

<220>
<223> conjugate

<400> 33
catatgagtt gcaacggccg ttgcggcgtc agatcatctt ctcgaacccc gagtgacaag 60

cctgtagccc atgttgtagc aaaccctcaa gctgaggggc agctccagtg gctgaaccgc 120

cgggccaatg ccctcctggc caatggcgtg gagctgagag ataaccagct ggtggtgcca 180

tcagagggcc tgtacctcat ctactcccag gtcctcttca agggccaagg ctgcccctcc 240

acccatgtgc tcctcaccca caccatcagc cgcatcgccg tctcctacca gaccaaggtc 300

aacctcctct ctgccatcaa gagcccctgc cagagggaga ccccagaggg ggctgaggcc 360

aagccctggt atgagcccat ctatctggga ggggtcttcc agctggagaa gggtgaccga 420

ctcagcgctg agatcaatcg gcccgactat ctcgactttg ccgagtctgg gcaggtctac 480

tttgggatca ttgccctgtg aggatcc 507

<210> 34
<211> 566
<212> DNA
<213> Artificial Sequence

<220>
<223> conjugate

<400> 34
ggatccaaac gatgagattc ccttctattt tcactgctgt tttgttcgct gcttcttctg 60

ctttggcttg taacggtaga tgtggtgtta gatcttcttc tagaactcct tctgataagc 120

ctgttgctca tgttgttgct aaccctcaag ctgaaggtca attgcaatgg ttgaacagaa 180

gagctaatgc tttgttggct aatggtgttg aattgagaga taatcaattg gttgttccat 240

ctgaaggttt gtacttgatc tattctcaag ttttgttcaa gggtcaaggt tgtccttcta 300

ctcatgtttt gttgactcat actatttcta gaatcgctgt ttcttatcaa actaaggtta 360

atttgctgtc tgctattaag tctccatgtc aaagagaaac tcctgaaggt gctgaagcta 420

agccatggta cgaaccaatc tatttgggtg gtgtttttca attggaaaag ggtgacagat 480

tgtctgctga aattaataga ccagattatt tggatttcgc tgaatctggt caagtttact 540

ttggtattat tgctttgtaa gaattc 566

<210> 35
<211> 4
<212> PRT
<213> Homo sapiens

<400> 35

Asp Gly Arg Cys
1

<210> 36
<211> 5
<212> PRT
<213> Homo sapiens

<220>
<221> MOD_RES
<222> (1)..(1)
<223> acetyl-Ciso

<400> 36

Asp Gly Arg Cys Lys
1               5

<210> 37
<211> 15
<212> PRT
<213> Mus musculus

<400> 37

Cys Asn Gly Arg Cys Gly Leu Arg Ser Ser Ser Gln Asn Ser Ser
1               5                   10                  15

<210> 38
<211> 6
<212> PRT
<213> Homo sapiens

<400> 38

Cys Asp Gly Arg Cys Gly
1               5

<210> 39

<211>  16
<212>  PRT
<213>  Homo sapiens

<400>  39

Cys Asn Gly Arg Cys Gly Leu Arg Ser Ser Ser Gln Asn Ser Ser Asp
1               5                   10                  15


<210>  40
<211>  28
<212>  PRT
<213>  Homo sapiens

<400>  40

Met Arg Ala Lys Leu Leu Gly Ile Val Leu Thr Thr Pro Ile Ala Ile
1               5                   10                  15


Ser Ser Phe Ala Ser Thr Cys Asn Gly Arg Cys Gly
            20                  25


<210>  41
<211>  4
<212>  PRT
<213>  Artificial Sequence

<220>
<223>  sequence


<220>
<221>  MOD_RES
<222>  (1)..(1)
<223>  Ciso or acetyl-Ciso or iso

<400>  41

Asp Gly Arg Cys
1


<210>  42
<211>  9
<212>  PRT
<213>  Artificial Sequence

<220>
<223>  sequence


<220>
<221>  MOD_RES
<222>  (1)..(1)
<223>  acetylated

<400>  42

Cys Asn Gly Arg Cys Gly Val Arg Tyr
1               5

<210>  43
<211>  25
<212>  PRT
<213>  Artificial Sequence

<220>
<223>  sequence

<400>  43

Met Arg Phe Pro Ser Ile Phe Thr Ala Val Leu Phe Ala Ala Ser Ser
1               5                   10                  15

Ala Leu Ala Cys Asn Gly Arg Cys Gly
            20                  25

## Description

### TECHNICAL FIELD

The present invention refers to a conjugate comprising a first peptide of sequence CNGRCG (SEQ ID NO: 1) linked to the N-terminus of a protein, e.g. TNF, and a compound X, e.g. serine, linked to the N-terminus of said peptide and to their therapeutic use. The invention also refers to methods for producing homogenous conjugate comprising a first peptide of sequence CNGRCG (SEQ ID NO: 1) linked to the N-terminus of a protein.

### BACKGROUND ART

The efficacy of cytokines in cancer therapy is often limited by systemic toxicity and counter-regulatory mechanisms. Recent studies suggest that these limitations can be overcome by targeting strategies based on the conjugation of these proteins with ligands capable of delivering them to the tumor site, thereby allowing administration of lower doses and reducing systemic effects (Corti and Curnis 2011; Corti et al. 2013). Among the various approaches so far developed, cytokine conjugation or fusion with antibodies or peptide ligands capable of recognizing specific receptors in tumor tissues are the most advanced. These ligands typically recognize receptors expressed by tumor cells or elements of the tumor microenvironment, including the tumor vasculature. A prototypic example of this concept is a drug based on the conjugation of a peptide ligand containing the CNGRCG (SEQ ID NO: 1) sequence, which recognize CD13-positive tumor vessels, and tumor necrosis factor-α (TNF), a cytokine capable of altering the endothelial barrier function and promoting chemotherapeutic drug penetration/ immune cell infiltration in tumor tissues (Corti and Curnis 2011; Corti et al. 2013). This drug, which was prepared by recombinant DNA technology in the early 2000s and which represents the first peptide-cytokine conjugate developed, is being tested in several clinical trials on cancer patients with evidence of activity (Corti et al. 2013; Ferreri et al. 2019). A major limitation of the CNGRCG-TNF drug (called hereinafter NGR-TNF) and of other CNGRCG-cytokine conjugates is related to the instability of the CNGRCG (SEQ ID NO: 1) motif and to its heterogeneity, owing to unwanted modification reactions, which rise serious problems in drug manufacturing and storage and which may have important pharmacological and toxicological implications that need to be clarified for drug registration (Corti et al. 2013).

### The NGR motif

The NGR motif has been discovered in the nineties by in vivo selection of peptide-phage libraries in tumor-bearing mice (Arap, Pasqualini, and Ruoslahti 1998; Corti et al. 2008). Systemic administration of a phage library into nude mice bearing human breast carcinoma xenografts led to the selection of tumor vasculature-homing phages carrying various peptide sequences containing this motif. Mechanistic studies showed that a cyclic disulfide-bridged peptide containing NGR (CNGRC (SEQ ID NO:5)) can specifically recognize vessels expressing aminopeptidase N (CD13), a membrane-bound metalloproteinase that is barely or not at all expressed by normal blood vessels, but is up-regulated in angiogenic blood vessels (Curnis et al. 2002; Pasqualini et al. 2000; Lahdenranta et al. 2007; Buehler et al. 2006). This protease has a role in protein degradation, cytokine regulation, antigen presentation, cell proliferation, cell migration, and angiogenesis (Curnis et al. 2002; Mina-Osorio 2008; Luan and Xu 2007; Bhagwat et al. 2001). In tumor tissues, CD13 is expressed by endothelial cells and pericytes, and, in some cases, by tumor cells and fibroblasts. CD13 is also expressed by many cells of normal tissues, including epithelial cells from the small intestine, proximal renal tubules, prostate, bile duct canaliculi, keratinocytes, mast cells, myeloid cells, and antigen-presenting cells (Curnis et al. 2002; Taylor 1993; Shipp and Look 1993; Dixon et al. 1994; Di Matteo et al. 2010). Immunohistochemical and biodistribution studies showed that CNGRC-containing compounds bind CD13-positive tumor blood vessels, but not other CD13-rich tissues (Curnis et al. 2002; Curnis et al. 2000). Direct binding assays with a CNGRCG-TNF conjugate (NGR-TNF) and competitive binding assays with anti-CD 13 antibodies showed that a CD13 form expressed by tumor blood vessels could function as a vascular receptor for the NGR motif. In contrast, CD13 expressed in normal kidney and in myeloid cells failed to bind to NGR-TNF. Consistently with these results, neither radiolabeled ¹²⁵I-NGR-TNF nor ¹²⁵I-TNF accumulated in normal organs containing CD13-expressing cells after administration to mice (Curnis et al. 2002). It would appear, therefore, that a functionally active form of CD13, in terms of NGR binding, is present in the tumor vasculature, but not in other CD13-rich tissues. The structural basis of this NGR selectivity is still unknown. The recognition of angiogenic blood vessels by NGR has been demonstrated also with cyclic-NGR-labeled paramagnetic quantum dots and quantitative molecular magnetic resonance imaging in tumor mouse models (Oostendorp et al. 2008). Ex-vivo two-photon laser scanning microscopy showed that these particles bind primarily to the endothelial lining of tumor vessels.

### Use of NGR-peptides as drug-delivery systems

Peptides containing the NGR sequence have been used by several investigators for delivering a variety of compounds to tumor blood vessels, including chemotherapeutic drugs, liposomes, anti-angiogenic compounds, DNA complexes, viral particles, and imaging compounds (Corti and Curnis 2011; Corti et al. 2013). NGR peptides have been also fused to cytokines, such TNFα, IFNγ and IFNα-2a, in an attempt to improve their anti-tumor therapeutic index (Corti and Curnis 2011; Corti et al. 2013). Thus, a variety of different compounds have been coupled to NGR peptides by different investigators, with good results. It is noteworthy that these products rely on the use of various peptides having NGR embedded in different molecular scaffolds, such as the prototypic disulfide-bridged CNGRC (SEQ ID NO:5), acetylated-CNGRC, CVLNGRMEC (SEQ ID NO:12), head-to-tail cyclized KNGRE (SEQ ID NO:7), CGNGRG (SEQ ID NO:8), or linear GNGRG (SEQ ID NO:9), NGRAHA (SEQ ID NO:10), KNGRE (SEQ ID NO:7), NGR and several others (Corti and Curnis 2011; Corti et al. 2008). These peptides have been chemically coupled to drugs and particles or fused to the N-terminal or C-terminal sequences of proteins, or even incorporated in internal loops of proteins by genetic engineering technology (Corti and Curnis 2011; Corti et al. 2008). The good results obtained with many of these products highlight the utility and versatility of NGR as a targeting motif for drug development. However, the CNGRC (SEQ ID NO:5) peptide may represent the preferred choice for drug development, because it has a proven utility for drug delivery to the tumor vasculature in patients (including the vasculature in the brain tumors) and has a documented poor immunogenicity in animals and patients. The advanced stage of development of CNGRC (SEQ ID NO:5) as a ligand in patients may represent, therefore, an important advantage over the others.

### Pharmacological and toxicological properties of NGR-TNF in animal models and in cancer patients

Administration of ultra-low dose (picograms) of murine CNGRCG-TNF (NGR-TNF), but not of murine TNF, exerts synergistic antitumor effects with various chemotherapeutic drugs, such as doxorubicin, melphalan, cisplatin, paclitaxel and gemcitabine, in various animal models of melanoma, prostate cancer, lymphoma, fibrosarcoma, and mammary adenocarcinoma, by altering drug-penetration barriers (Curnis, Sacchi, and Corti 2002; Sacchi et al. 2006; Curnis et al. 2000). Inhibition of tumor growth in melanoma and lymphoma animal models have been observed also with intramuscular injection of plasmid DNA encoding for NGR-TNF, but not plasmid DNA encoding for TNF alone (Zarovni, Monaco, and Corti 2004). These studies have also shown that CNGRCG-mediated vascular-targeting of TNF is a valuable strategy for delivering bioactive amounts of cytokine to tumor endothelial cells without causing the activation of counter-regulatory mechanisms and toxic reactions (Curnis, Sacchi, and Corti 2002; Corti et al. 2013). Furthermore, these studies have shown that targeted delivery of minute amounts of TNF to tumor vessels is sufficient to alter the endothelial barrier function and favor not only the penetration of chemotherapeutic drugs in tumors, but also the infiltration of lymphocytes in neoplastic tissues (Curnis, Sacchi, and Corti 2002; Sacchi et al. 2006; Calcinotto et al. 2012). Indeed, low-dose NGR-TNF (0.1 ng) can promote lymphocyte extravasation in tumors by up-regulating leukocyte adhesion molecules on tumor vessels and by inducing the release of various chemokines in tumor tissues (Calcinotto et al. 2012). These mechanisms associate with increased tumor infiltration of endogenous or adoptively transferred cytotoxic T lymphocytes in transplantable models of melanoma and in the TRAMP model of spontaneous prostate cancer, without modification of T-cell distribution in blood, spleen or kidney of tumor-bearing mice (Calcinotto et al. 2012), and increased overall survival of tumor-bearing mice with no evidence of toxicity. NGR-TNF can also increase the efficacy of active immunotherapy (vaccination) either alone or in combination with chemotherapy (Calcinotto et al. 2012). Because of these properties a human version of NGR-TNF consisting of CNGRCG (SEQ ID NO: 1) fused to the human TNF sequence has been tested in various Phase II and III clinical studies in patients with solid tumors, alone and in combination with chemotherapy or immunotherapy, with evidence of activity (www.molmed.com). The biological and pharmacological properties of this product and the results of phase I and II clinical studies have been reviewed (Corti et al. 2013). These studies showed that NGR-TNF is well tolerated. Chills and fever were the most frequently observed toxicities and no patients developed anti-NGR-TNF antibodies during treatment. Dynamic contrast-enhanced magnetic resonance imaging showed a vascular response to NGR-TNF. Single-agent phase II studies with low-dose NGR-TNF (0.8 µg/m², 1 h infusion, every three weeks or weekly), conducted in malignant pleural mesothelioma (MPM), hepatocellular carcinoma and colorectal cancer, showed radiological anti-vascular effects and significant disease control. In particular, a phase II study on MPM patients showed disease control in about half of previously treated patients, which was maintained in the triweekly cohort for 4.4 months and for 9.1 months in the weekly cohort (Gregorc et al. 2010). Based on these results, a randomized double-blind phase III study of human NGR-TNF plus best investigator's choice (called "BIC") versus placebo plus BIC in previously treated patients with advanced MPM have been undertaken. The results have shown a significant increase of the overall survival in patients with more aggressive MPM (Gregorc et al. 2018). These results are remarkable considering that, currently, there are no standard options for patients with MPM who are failing a front-line pemetrexed-based regimen and also considering the easily manageable toxicity profile of NGR-TNF. Phase I and II studies of human NGR-TNF in combination with chemotherapy (e.g. doxorubicin or cisplatin) in patients with refractory solid tumors showed that this drug combination has interesting clinical activity and safe toxicity profile (Zucali et al. 2013; Lorusso et al. 2012; Gregorc et al. 2011; Corti et al. 2013). Finally, a recent Phase II study performed in patients with relapsing/refractory primary central nervous system lymphoma (PCNSL) have shown that NGR-TNF can alter the blood brain barrier in the tumors and increase the efficacy of R-CHOP, leading to 75% of responses (50% complete) (Ferreri et al. 2019). These results, overall, indicate that NGR-TNF is a biologically active and safe molecule with a high potential to be translated into a commercially available drug.

### Molecular heterogeneity and stability of recombinant NGR-TNF

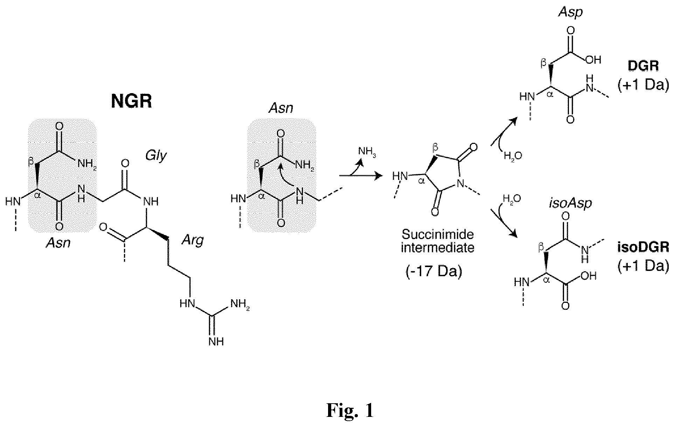

In principle NGR-TNF is a homogeneous 50 kDa homotrimeric protein. Biochemical characterization studies of human NGR-TNF have shown that this drug is indeed a trimeric protein, but composed by different subunits, including: a) subunits with a molecular weight consistent with the expected value (+0Da), b) subunits characterized by a 17 Da smaller molecular weight (-17Da), c) subunits characterized by 42 Da larger molecular weight (+42Da), d) subunits characterized by 58 Da larger molecular weight (+58Da) (Tobias et al. 2013). Considering that subunits can associate in a random manner to form trimers inventors estimate that at least 64 (4³) different trimers may exist in human NGR-TNF preparations. The heterogeneity of this drugs may further increase considering that the asparagine (N) of the NGR motif may undergo rapid deamidation. Indeed, several studies have shown that asparagine of CNGRC (SEQ ID NO:5) deamidates very rapidly (half-life 4-5 h in physiological buffer) via succinimide intermediate (characterized by loss of 17 Da), which upon hydrolytic cleavage leads to formation of Asp (D) and *iso*Asp (isoD) in a 1:3 ratio in L or D configurations, both characterized by the gain of 1 Da. Thus, the deamidation of CNGRC (SEQ ID NO:5) generates CDGRC (SEQ ID NO:11) and C*iso*DGRC (SEQ ID NO:41) sequences (Corti and Curnis 2011; Curnis et al. 2010; Curnis et al. 2006) (see Fig. 1 for a schematic representation). It is therefore conceivable that an unpredictable number of different NGR-TNF variants can be present in NGR-TNF and/or maybe formed upon storage or after injections to patients. Remarkably, CNGRC (SEQ ID NO:5) deamidation causes loss of CD13 binding affinity and gain of integrin binding properties. Indeed, the CisoDGRC product, but not CDGRC (SEQ ID NO:11), can bind the RGD-binding pocked of αvβ3 (Corti and Curnis 2011; Curnis et al. 2010; Curnis et al. 2006; Spitaleri et al. 2008), an integrin overexpressed in the tumor neovasculature. Notably, the affinity and specificity of C*iso*DGRC for αvβ3 and other integrins strongly depend on flanking residues and even small changes can have a dramatic effect. For example, while C*iso*DGRC can bind αvβ3 with an affinity 10-100-fold greater than αvβ5, αvβ6, αvβ8, and α5β1, the acetyl-C*iso*DGRC (SEQ ID NO:41) peptide (+42Da) can bind αvβ3, αvβ6, and α5β1 with similar affinities (Curnis et al. 2010). Thus, the pharmacological and toxicological properties of each compound potentially present or formed in NGR-TNF could be different because of the different affinity for CD 13 or for integrins that each form may have. The various forms present in NGR-TNF (-17Da, +0Da, +42Da, +58Da and the deamidated corresponding forms) are likely related to modifications occurring during NGR-TNF expression in *E.coli* cells, its purification, its storage, and, possibly (albeit in small amounts) after administration in patients. It is therefore obvious that a) biochemical, biological, pharmacological and toxicological properties of each component have to be carefully defined before drug registration and b) reproducible composition of different NGR-TNF production lots should be guaranteed for their use in patients. Both these tasks maybe very difficult, considering the complexity of this drug.

Thus, the development of methods for the production of homogeneous NGR-TNF devoid of post-translational modifications, or the identification of a single-component, stable, and bioactive derivative of NGR-TNF is highly desirable. There is therefore a need for targeted cytokines derivatives for treating cancers, which are stable and homogenous.

### SUMMARY OF THE INVENTION

A growing body of evidence suggests that the efficacy of cytokines in cancer therapy can be increased by targeting strategies based on conjugation with peptides containing the NGR motif, i.e. with ligands that recognize CD13-positive tumor blood vessels. The targeting approach is generally conceived to permit administration of low, yet pharmacologically active doses of drugs, thereby avoiding toxic reactions and activation of systemic counter-regulatory mechanisms. In particular, the CNGRCG (SEQ ID NO:1) peptide has been used to produce, by recombinant DNA technology, the CNGRCG-tumor necrosis factor-α (TNF) fusion protein, which is currently used in clinical trials on cancer patients for various indications. A major limitation of CNGRCG-TNF (called NGR-TNF) and other CNGRCG-cytokine products is related to the instability of the CNGRCG (SEQ ID NO:1) motif and to its molecular heterogeneity, owing to undesirable post-translational modification reactions, which rise serious problems and difficulties in drug manufacturing and storage, and which may also have potential implications in drug pharmacology and toxicology. The present invention is related to CNGRCG-cytokine conjugates (X-CNGRCG-cytokine) that are more stable and more homogeneous than the CNGRCG-cytokine previously developed, thereby representing a more reliable second-generation-targeted cytokine.

Therefore, the invention provides a conjugate comprising:
- a first peptide of sequence CNGRCG (SEQ ID NO:1) linked to the N-terminus of a protein;
- a compound X linked to the N-terminus of said peptide.

Preferably, the conjugate is able to recognize CD13.

In the conjugate according to the invention the protein is preferably a cytokine, more preferably a cytokine endowed of anti-tumor activity. The cytokine is preferably selected from the group consisting of: tumor necrosis factor (TNF), preferably TNF-alpha or TNF-beta, TNF-related apoptosis inducing ligand (TRAIL), endothelial monocyte activating polypeptide II (EMAP-II), IL12, IFNgamma and IFNalpha.

Preferably the compound X is a second peptide of 1-200 amino acid residues, more preferably of 1, 2 or 3 amino acid residues.

Preferably, the second peptide consists of: a serine residue or any amino acid with a short side chain, preferably glycine or alanine, an amino acid sequence comprising the IEGR (SEQ ID NO: 2) sequence or a leader sequence which is removed upon expression of the conjugate in eukaryotic cells and secretion, preferably an OmpT leader sequence.

Preferably the second peptide consists of a serine and the cytokine is TNF. Preferably TNF is human TNF-alpha.

In the conjugate according to the invention the conjugate contains a site for chemical or enzymatic cleavage of the bond between the compound X and the peptide (X-C bond), preferably wherein the cleavage of the X-C bond can be achieved with an aminopeptidase or an endoprotease, preferably with aminopeptidase N (CD13) or with a protease, preferably factor Xa.

Further objects of the invention are a nucleic acid encoding for the conjugate as above defined, a vector, preferably a plasmid or a viral vector, for gene therapy containing the said nucleic acid, and a nanoparticle comprising the conjugate as above defined, preferably the conjugate is adsorbed on the surface of gold nanoparticles.

Another object of the invention is a combination product comprising the conjugate as above defined or the nucleic acid as above defined or the vector as above defined or the nanoparticle as above defined and at least one antitumor agent, preferably being a chemotherapeutic agent and/or immunomodulator and/or autoimmune cell. Preferably the chemotherapeutic agent is doxorubicin, melphalan, gemcitabine, taxol, cisplatin, vincristine, or vinorelbine. Preferably the immunomodulator is an anticancer vaccine or an immune check point blocker, such as anti-PD1 or anti-PDL1 or antiCTLA4 antibodies. Preferably the immune cell is a lymphocyte or a genetically modified T-lymphocyte, such as CAR-T cells, or TCR redirected T-cells or NK cells. Preferably the further antitumor agent comprises an antibody and a chemotherapeutic agent, such as R-CHOP: rituximab, cyclophosphamide, vincristine, doxorubicin, prednisolone.

The conjugate or the combination product as above defined are preferably for medical use, more preferably for use the treatment of tumors, preferably solid tumors, more preferably brain tumors (e.g. glioblastoma and astrocytoma), lymphomas, preferably primary diffuse large B-cell lymphoma of the CNS (PCNSL), sarcoma, melanoma oral or skin squamous cell carcinoma, hepatocellular carcinoma, head and neck, gastroesophageal, colorectal, pancreatic, ovarian, lung, cervix, breast cancer, renal, urothelial or metastasis thereof.

Preferably at least one antitumor agent as defined above is also administered. In the treatment of primary diffuse large B-cell lymphoma of the CNS (PCNSL), R-CHOP is also preferably administered.

Another object of the invention is a pharmaceutical composition comprising an effective amount of a conjugate or the nucleic acid or the vector or the nanoparticle as above defined, together with at least one pharmaceutically acceptable carrier and/or excipient. Optionally the pharmaceutical composition further comprises at least one antitumor agent.

A further object of the invention is a method for producing a homogeneous conjugate comprising the sequence CNGRCG (SEQ ID NO: 1) linked to the N-terminus of a protein, said method comprising:
the expression of a conjugate as defined above in prokaryotic or eukaryotic cells, preferably *E.coli* cells and *B. subtilis;*
chemical or enzymatic cleavage of X-C bond, preferably with an aminopeptidase, an endoproteinase or a protease.

Another object of the invention is a method for the production of a homogeneous conjugate comprising the sequence CNGRCG (SEQ ID NO: 1) linked to the N-terminus of a protein, said method comprising DNA expression in a host unable to acetylate the alpha-amino group or to modify the CN sequence.

A further object of the invention is a method for the production of a homogeneous conjugate comprising the sequence CNGRCG (SEQ ID NO: 1) linked to the N-terminus of a protein, said method comprising:
the expression of said conjugate further comprising a leader sequence which is removed upon expression in eukaryotic cells or plant or animals, preferably in *Pichia Pastoris* cells, CHO cells, baculovirus insect cells systems,
secretion of the conjugate.

The cytokine is preferably an inflammatory cytokine. In one preferred embodiment the cytokine is a therapeutic cytokine. Preferably the cytokine is TNFα, TNFβ, IFNα, IFNβ, IFNγ, IL-1, 2, 4, 6, 12, 15, 18, EMAP II, vascular endothelial growth factor (VEGF), PDGF, PD-ECGF or a chemokine. In one embodiment the cytokine is TNF-α, TNF-β or IFN-γ.

Preferably the conjugate is in the form of a fusion protein. In another embodiment the conjugate is in the form of nucleic acid, a plasmid or a viral vector for gene therapy. In another embodiment the conjugate is in the form of a nanoparticle, e.g. adsorbed on the surface of gold nanoparticles
In a preferred embodiment the composition further comprises another antitumor agent.

Preferably the further antitumor agent is a chemotherapeutic drug, or an immunomodulator, or immune cells. Preferably the chemotherapeutic drug is doxorubicin, melphalan, gemcitabine, taxol, cisplatin, vincristine, or vinorelbine. Preferably the immunomodulator is an anticancer vaccine or an immune check point blocker (such as anti-PD1 or anti-PDL1 or antiCTLA4 antibodies). Preferably immune cells are lymphocytes or genetically modified T-lymphocytes (such as CAR-T cells, or TCR redirected T-cells). Preferably the further antitumor agent comprises an antibody and a chemotherapeutic agent (such as R-CHOP: rituximab, cyclophosphamide, vincristine, doxorubicin, prednisolone)

Other objects of the invention are an expression vector comprising the nucleic acid as defined above, a host cell transformed with said expression vector and a method for preparing a conjugate or fusion protein as defined above comprising culturing said host cell under conditions which provide for the expression of the conjugate or of the fusion protein.

### DETAILED DESCRIPTION OF THE INVENTION

Inventors herein disclose novel methods for the production of homogeneous CNGRCG-cytokine conjugates (including NGR-TNF) and novel CNGRCG-cytokine derivatives that are more stable, homogeneous and bioactive than those originally described. Inventors have found that an important source of molecular heterogeneity and instability of the CNGRCG (SEQ ID NO:1) domain coupled to cytokines, such as TNF and EMAP-II, is related to the presence of a cysteine followed by an asparagine residue (CN) in the N-terminus of NGR-TNF. Furthermore, inventors have discovered that changing the N-terminal sequence of NGR-TNF by adding an additional aminoacid or a polypeptide sequence improves drug stability and overcomes the problem of drug heterogeneity. In a preferred embodiment of the present invention the problem of molecular heterogeneity is solved by adding a serine residue to the N-terminus of NGR-TNF (SCNGRCG-TNF, called S-NGR-TNF), a product that is more stable and homogeneous than the conventional NGR-TNF (CNGRCG-TNF). This product can bind CD13 with improved affinity and is endowed with potent antitumor activity in animal models either alone or in combination with chemotherapy. Thus, S-NGR-TNF represents a new molecule with improved biochemical and biological properties compared to NGR-TNF. It is obvious for an expert in the art that the same strategy could be applied also for the generation of other CNGRCG-cytokine conjugates, to solve the problem of CN instability and molecular heterogeneity. It is also obvious for an expert in the art that the N-terminus of CNGRCG-TNF or other CNGRCG-cytokine conjugates could be modified with an aminoacid different from serine or even with a di-, tri- or multi-residues peptide, to improve drug stability and homogeneity. Hereinafter, the extra sequence fused to CNGRCG-cytokine is called "X" sequence, thereby generating an X-CNGRCG-cytokine (for example the X-CNGRCG-TNF).

In another preferred embodiment of the invention the problem of molecular heterogeneity of CNGRCG-cytokine conjugates is solved by fusing CNGRCG (SEQ ID NO:1) with the C-terminus of an X polypeptide sequences that is cleaved upon expression in appropriate system, such as the OmpT leader sequence for periplasmatic expression in E.coli cells, or other leader sequence that are removed upon expression in yeast or other eukaryotic cells and secretion.

Given these premises, it is also obvious for an expert in the art that an alternative method for the preparation of homogeneous CNGRCG-cytokine (including NGR-TNF) could rely on the expression of X-CNGRCG-cytokine followed by chemical or enzymatic removal of the X sequence in vitro. For example, aminopeptidases or other endoproteinases capable of cleaving the X-C peptide bond in the X-CNGRCG sequence could be exploited to remove the X moiety leaving the CNGRCG-cytokine with no chemical modification of its N-terminus. For example, aminopeptidase N could be exploited when X is a leucine or an alanine residue, whereas factor Xa could be used when X is a tag containing the IEGR sequence fused to CNGRCG (SEQ ID NO: 1).

Another possibility to generate homogeneous CNGRCG-cytokine products is to express them in organisms that cannot modify the CN sequence, such as eukaryotic cells, or to express conjugates with peptides lacking the N-terminal cystine, such as NGR followed by other amino acid residues such as or NGRAHA (SEQ ID NO:10) or NGRAGG (SEQ ID NO: 13).

The present invention relates to a conjugate which is a molecule comprising at least one targeting moiety/polypeptide linked to at least one cytokine formed through genetic fusion or chemical coupling. By "linked" we mean that the first and second sequences are associated such that the first sequence is able to be transported by the second sequence to a target cell. Thus, conjugates include fusion proteins in which the transport protein is linked to a cytokine via their polypeptide backbones through genetic expression of a DNA molecule encoding these proteins, directly synthesized proteins and coupled proteins in which pre-formed sequences are associated by a cross-linking agent. The term is also used herein to include associations, such as aggregates, of the cytokine with the targeting peptide/protein. The conjugates of the present invention are capable of being directed to a cell so that an effector function corresponding to the polypeptide sequence coupled to the transport sequence can take place. The peptide can be coupled directly to the cytokine or indirectly through a spacer, which can be a single amino acid, an amino acid sequence or an organic residue, such as 6-aminocapryl-N-hydroxysuccinimide.

The peptide ligand is preferably linked to the cytokine N-terminus thus minimising any interference in the binding of the modified cytokine to its receptor. Alternatively, the peptide can be linked to amino acid residues which are amido- or carboxylic-bond acceptors, which may be naturally occurring on the molecule or artificially inserted using genetic engineering techniques. The modified cytokine is preferably prepared by use of a cDNA comprising a 5 '-contiguous sequence encoding the peptide. According to a preferred embodiment, there is provided a conjugation product between TNF and the CNGRC (SEQ ID NO:5) sequence in which the amino-terminal of TNF is linked to the CNGRC (SEQ ID NO:5) peptide through the spacer G (glycine). The conjugate also comprises a compound X as defined above.

The cDNA coding for the conjugate of the present invention, or for the protein and/or for the compound X may be codon optimized for the expression in the host.

Chemotherapeutic drug penetration into neoplastic cells is critical for the effectiveness of solid-tumor chemotherapy. To reach cancer cells in solid tumors, chemotherapeutic drugs must enter the drug blood vessels, cross the vessel wall and finally migrate through the interstitium. Heterogeneous tumor perfusion, vascular permeability and cell density, and increased interstitial pressure may represent critical barriers that may limit the penetration of drugs into neoplastic cells and, consequently, the effectiveness of chemotherapy.

The same applies for anticancer immunomodulators or immune cells, which also need to penetrate in tumor tissues for their action. Cytokines which have the effect of affecting these factors and that can alter drug penetration barriers are therefore useful in the present invention. A non-limiting list of cytokines which may be used in the present invention is: TNFα, TNFβ, IFNα, IFNβ, IFNγ, IL-1, 2, 4, 6, 12, 15, EMAP II, vascular endothelial growth factor (VEGF), PDGF, PD-ECGF or a chemokine. In one embodiment the TNF is a mutant form of TNF capable of selectively binding to one of the TNF receptors (Loetscher H et al (1993) J Biol Chem 268:26350-7; Van Ostade X et al (1993) Nature 361 :266-9). In another embodiment of the invention the TNF is mutant with a lower affinity for the TNF receptors (Huyghe et al, EMBO Molecular Medicine 12: e11223, 2020).

The invention is illustrated by the following examples related to TNF (TNF-alpha) and endothelial monocyte activating polypeptide II (EMAP-II). However, considering the high similarity of structure and anti-tumor activity of TNF-alpha with those of TNF-beta (also called lymphotoxin-alpha) and of TNF-related apoptosis inducing ligand (TRAIL), similar compounds based on TNF-beta or TRAIL could be easily generated by a skilled man.

Preferably, the conjugate according to the invention comprises from the N-terminus to the C-terminus: the compound X as above defined, the peptide of sequence CNGRCG (SEQ ID NO: 1), the protein as above defined. Preferably, the conjugate according to the invention comprises from the N-terminus to the C-terminus: a serine residue, the sequence CNGRCG (SEQ ID NO: 1), TNF-alpha. Preferably, the conjugate according to the invention comprises from the N-terminus to the C-terminus: the alpha mating factor secretion signal peptide, the sequence CNGRCG (SEQ ID NO: 1), TNF-alpha. Preferably, the conjugate according to the invention comprises from the N-terminus to the C-terminus: OmpT leader sequence, the sequence CNGRCG (SEQ ID NO: 1), TNF-alpha.

For "homogeneous" it is intended with the expected molecular weight.

The above first peptide of sequence CNGRCG (SEQ ID NO:1) may be directly linked to the N-terminus of a protein or linked through a linker. The above compound X may be directly linked to the N-terminus of said peptide or may be linked though a linker. E.g. conjugate of the invention may comprise between the above leader sequence and the first peptide of sequence CNGRCG (SEQ ID NO:1) a sequence comprising a restriction site.

Preferably the human TNF comprises or consists of the sequence:






 (SEQ ID NO: 14)

Preferably the murine TNF comprises or consists of the sequence:

L R S S S Q N S S D K P V A H V V A N H Q V E E Q L E W L
S Q R A N A L L A N G M D L K D N Q L V V P A D G L Y L
V Y S Q V L F K G Q G C P D Y V L L T H T V S R F A I S Y
Q E K V N L L S A V K S P C P K D T P E G A E L K P W Y E
P I Y L G G V F Q L E K G D Q L S A E V N L P K Y L D F A
E S G Q V Y F G V I A L (SEQ ID NO: 15)

Preferably the OmpT signal peptide comprises or consists of the sequence:
M R A K L L G I V L T T P I A I S S F A (SEQ ID NO: 16)

Preferably alpha mating factor secretion signal peptide comprises or consists of the sequence:
M R F P S I F T A V L F A A S S A L A (SEQ ID NO:23)

More preferably the conjugate according to the invention comprises or consists of the sequence 
MRAKLLGIVLTTPIAISSFASTCNGR C G L R S S S Q N S S D
K P V A H V V A N H Q V E E Q L E W L S Q R A N A L L A
N G M D L K D N Q L V V P A D G L Y L V Y S Q V L F K G
Q G C P D Y V L L T H T V S R F A I S Y Q E K V N L L S A
V K S P C P K D T P E G A E L K P W Y E P I Y L G G V F Q
L E K G D Q L S A E V N L P K Y L D F A E S G Q V Y F G V
I A L (SEQ ID NO: 24)
 or 
M S C N G R C G L R S S S Q N S S D K P V A H V V A N H Q
V E E Q L E W L S Q R A N A L L A N G M D L K D N Q L V
V P A D G L Y L V Y S Q V L F K G Q G C P D Y V L L T H T
V S R F A I S Y Q E K V N L L S A V K S P C P K D T P E G
A E L K P W Y E P I Y L G G V F Q L E K G D Q L S A E V N
L P K Y L D F A E S G Q V Y F G V I A L (SEQ ID NO: 25)
 or 
S C N G R C G L R S S S Q N S S D K P V A H V V A N H Q V
E E Q L E W L S Q R A N A L L A N G M D L K D N Q L V V
P A D G L Y L V Y S Q V L F K G Q G C P D Y V L L T H T V
S R F A I S Y Q E K V N L L S A V K S P C P K D T P E G A
E L K P W Y E P I Y L G G V F Q L E K G D Q L S A E V N L
P K Y L D F A E S G Q V Y F G V I A L (SEQ ID NO: 26)
 or 
M S C N G R C G V R S S S R T P S D K P V A H V V A N P Q
A E G Q L Q W L N R R A N A L L A N G V E L R D N Q L V
V P S E G L Y L I Y S Q V L F K G Q G C P S T H V L L T H
T I S R I A V S Y Q T K V N L L S A I K S P C Q R E T P E G
A E A K P W Y E P I Y L G G V F Q L E K G D R L S A E I N
R P D Y L D F A E S G Q V Y F G I I A L (SEQ ID NO: 27)
 or 
S C N G R C G V R S S S R T P S D K P V A H V V A N P Q A
E G Q L Q W L N R R A N A L L A N G V E L R D N Q L V V
P S E G L Y L I Y S Q V L F K G Q G C P S T H V L L T H T I
S R I A V S Y Q T K V N L L S A I K S P C Q R E T P E G A E
A K P W Y E P I Y L G G V F Q L E K G D R L S A E I N R P
D Y L D F A E S G Q V Y F G I I A L (SEQ ID NO:28)
 or

MRFPSIFTAVLFAASSALAC N G R C G 






(SEQ ID NO:29).

In the method for producing a homogeneous conjugate according to the invention, the expression of the conjugate may be carried out in any expression system known to the expert in the art.

Examples of expression systems are prokaryotic systems, such as *Bacillus subtilis, E. coli, Bacillus megaterium, Lactoccos Lactis,* ... Examples of eukaryotic expression systems are yeast systems, as e.g. *Saccharomyces cerevisiae* and *P. Pastoris,* fungus systems, as e.g. *Aspergillus niger and oryzae,* insects sytems, mammalian systems, as e.g. HEK293 and CHO, transgenic plants or animals. All the expression systems mentioned in the publication Gomes et al., Advances in Animal and Veterinary Sciences, June 2016, 4(7):346-356 are herein incorporated by reference.

In the context of the present invention an amino acid with a short side chain is preferably an amino acid with a chain comprising 1 to 6 carbon atoms, preferably 1-3 carbon atoms.

A polynucleotide or nucleic acid described herein can be present in a vector. A vector is a replicating polynucleotide, such as a plasmid, phage, or cosmid, to which another polynucleotide may be attached so as to bring about the replication of the attached polynucleotide. Construction of vectors containing a polynucleotide of the invention employs standard ligation techniques known in the art. See, e.g., Sambrook et al, Molecular Cloning: A Laboratory Manual, Cold Spring Harbor Laboratory Press (1989). A vector can provide for further cloning (amplification of the polynucleotide), i.e., a cloning vector, or for expression of the polynucleotide, i.e., an expression vector. The term vector includes, but is not limited to, plasmid vectors, viral vectors, cosmid vectors, transposon vectors, and artificial chromosome vectors. Examples of viral vectors include, for instance, adenoviral vectors, adeno-associated viral vectors, lentiviral vectors, retroviral vectors, and herpes virus vectors. A vector may be replication-proficient or replication- deficient. A vector may result in integration into a cell's genomic DNA. Typically, a vector is capable of replication in a host cell, for instance a mammalian and/or a bacterial cell, such as E. coli.

Selection of a vector depends upon a variety of desired characteristics in the resulting construct, such as a selection marker, vector replication rate, use in gene transfer into cells of the gastrointestinal tract, and the like. Suitable host cells for cloning or expressing the vectors herein are prokaryotic or eukaryotic cells. Suitable eukaryotic cells include mammalian cells, such as murine cells and human cells. Suitable prokaryotic cells include eubacteria, such as gram- negative organisms, for example, E. coli.

An expression vector optionally includes regulatory sequences operably linked to the polynucleotide of the present invention. An example of a regulatory sequence is a promoter. A promoter may be functional in a host cell used, for instance, in the construction and/or characterization of CgA polynucleotide or a fragment thereof, and/or may be functional in the ultimate recipient of the vector. A promoter may be inducible, repressible, or constitutive, and examples of each type are known in the art. A polynucleotide of the present invention may also include a transcription terminator. Suitable transcription terminators are known in the art.

Polynucleotides described herein can be produced in vitro or in vivo. For instance, methods for in vitro synthesis include, but are not limited to, chemical synthesis with a conventional DNA/RNA synthesizer. Commercial suppliers of synthetic polynucleotides and reagents for in vitro synthesis are known. Methods for in vitro synthesis also include, for instance, in vitro transcription using a circular or linear expression vector in a cell free system. Expression vectors can also be used to produce a polynucleotide of the present invention in a cell, and the polynucleotide may then be isolated from the cell.

Also provided are compositions including one or more polypeptides or polynucleotides described herein. Such compositions typically include a pharmaceutically acceptable carrier. As used herein "pharmaceutically acceptable carrier" includes, but is not limited to, saline, solvents, dispersion media, coatings, antibacterial and antifungal agents, isotonic and absorption delaying agents, and the like, compatible with pharmaceutical administration. Additional compounds can also be incorporated into the compositions.

A composition may be prepared by methods known in the art of pharmacy. In general, a composition can be formulated to be compatible with its intended route of administration. A formulation may be solid or liquid. Administration may be systemic or local. In some aspects local administration may have advantages for site-specific, targeted disease management. Local therapies may provide high, clinically effective concentrations directly to the treatment site, with less likelihood of causing systemic side effects.

Examples of routes of administration include parenteral (e.g., intravenous, intradermal, subcutaneous, intraperitoneal, intramuscular), enteral (e.g., oral or rectal), and topical (e.g., epicutaneous, inhalational, transmucosal) administration. Appropriate dosage forms for enteral administration of the compound of the present invention may include tablets, capsules or liquids. Appropriate dosage forms for parenteral administration may include intravenous administration. Appropriate dosage forms for topical administration may include nasal sprays, metered dose inhalers, dry-powder inhalers or by nebulization. Solutions or suspensions can include the following components: a sterile diluent such as water for administration, saline solution, fixed oils, polyethylene glycols, glycerin, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates; electrolytes, such as sodium ion, chloride ion, potassium ion, calcium ion, and magnesium ion, and agents for the adjustment of tonicity such as sodium chloride or dextrose. pH can be adjusted with acids or bases, such as hydrochloric acid or sodium hydroxide. A composition can be enclosed in, for instance, ampoules, disposable syringes or multiple dose vials made of glass or plastic.

Compositions can include sterile aqueous solutions or dispersions and sterile powders for the extemporaneous preparation of sterile solutions or dispersions. For intravenous administration, suitable carriers include human albumin, physiological saline, bacteriostatic water, Cremophor EL™ (BASF, Parsippany, N.J.) or phosphate buffered saline. A composition is typically sterile and, when suitable for injectable use, should be fluid to the extent that easy syringability exists. It should be stable under the conditions of manufacture and storage and preserved against the contaminating action of microorganisms such as bacteria and fungi. The carrier can be a solvent or dispersion medium containing, for example, albumin, water, ethanol, polyol (for example, glycerol, propylene glycol, and liquid polyethylene glycol, and the like), and suitable mixtures thereof. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, sorbitol, and sodium chloride in the composition. Prolonged absorption of the injectable compositions can be brought about by including in the composition an agent which delays absorption, for example, aluminum monostearate and gelatin. Sterile solutions can be prepared by incorporating the active compound (e.g., a polypeptide or polynucleotide described herein) in the required amount in an appropriate solvent with one or a combination of ingredients such as those enumerated above, as required, followed by filtered sterilization. Generally, dispersions are prepared by incorporating the active compound into a sterile vehicle, which contains a dispersion medium and other ingredient such as from those enumerated above. In the case of sterile powders for the preparation of sterile injectable solutions, methods of preparation that may be used include vacuum drying and freeze- drying which yields a powder of the active ingredient plus any additional desired ingredient from a previously sterile-filtered solution thereof.

For enteral administration, a composition may be delivered by, for instance, nasogastric tube, enema, colonoscopy, or orally. Oral compositions may include an inert diluent or an edible carrier. For the purpose of oral therapeutic administration, the active compound can be incorporated with excipients and used in the form of tablets, troches, or capsules. Oral compositions can also be prepared using a fluid carrier. Pharmaceutically compatible binding agents can be included as part of the composition. The tablets, pills, capsules, troches and the like may contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the active compounds may be delivered in the form of an aerosol spray, a nebulizer, or an inhaler, such as a nasal spray, metered dose inhaler, or dry- powder inhaler. Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds may be formulated into ointments, salves, gels, or creams as generally known in the art. An example of transdermal administration includes iontophoretic delivery to the dermis or to other relevant tissues. The active compounds can also be prepared in the form of suppositories (e.g., with conventional suppository bases such as cocoa butter and other glycerides) or retention enemas for rectal delivery. The active compounds may be prepared with carriers that will protect the compound against rapid elimination from the body, such as a controlled release formulation, including implants. Biodegradable, biocompatible polymers can be used, such as ethylene vinyl acetate, polyanhydrides, polyglycolic acid, collagen, polyorthoesters, and polylactic acid. Such formulations can be prepared using standard techniques. The materials can also be obtained commercially. Liposomal suspensions can also be used as pharmaceutically acceptable carriers. These can be prepared according to methods known to those skilled in the art. Delivery reagents such as lipids, cationic lipids, phospholipids, liposomes, and microencapsulation may also be used.

### Sequences:

Included in the present invention are also nucleic acid sequences derived from the nucleotide sequences shown below, e.g. functional fragments, mutants, derivatives, analogues, and sequences having a % of identity of at least 70% with the below sequences.

The protein mentioned below are preferably characterized by the sequences disclosed by the corresponding NCBI accession numbers.

| **Protein** | **Accession number** | **Version number** |
|---|---|---|
| TNF-alpha | AQY77150 | AQY77150.1 |
| Lymphotoxin-alpha precursor | NP_001153212 | NP_001153212.1 |
| Endothelial-monocyte activating polypeptide | AAA62202 | AAA62202.1 |
| TNF-related apoptosis-inducing ligand | P50591 | P50591.1 |
| Interleukin-12 subunit beta precursor | NP_002178 | NP_002178.2 |
| Interleukin-12 subunit alpha isoform 1 precursor | NP_000873 | P50591.1 |
| Interferon gamma precursor | NP_000610 | NP_000610.2 |
| Interferon alpha-2 | P01563 | P01563.1 |
| Mating factor alpha-1 | P01149 | P01149.1 |
| Protease 7 | P09169 | P09169.1 |

### Murine NGR-TNF cloned in pET12 plasmid:

M R A K L L G I V L T T P I A I S S F A S T C N
CAT ATG CGG GCG AAA CTC CTA GGA ATA GTC CTG ACA ACC CCT ATC GCG ATC AGC TCT TTT GCG TCG ACG TGC AAC

G R C G L R S S S Q N S S D K P V A H V V A N H Q
GGC CGT TGT GGC CTC AGA TCA TCT TCT CAA AAT TCG AGT GAC AAG CCT GTA GCC CAC GTC GTA GCA AAC CAC CAA

V E E Q L E W L S Q R A N A L L A N G M D L K D N
GTG GAG GAG CAG CTG GAG TGG CTG AGC CAG CGC GCC AAC GCC CTC CTG GCC AAC GGC ATG GAT CTC AAA GAC AAC

Q L V V P A D G L Y L V Y S Q V L F K G Q G C P D
CAA CTA GTG GTG CCA GCC GAT GGG TTG TAC CTT GTC TAC TCC CAG GTT CTC TTC AAG GGA CAA GGC TGC CCC GAC

Y V L L T H T V S R F A I S Y Q E K V N L L S A V
TAC GTG CTC CTC ACC CAC ACC GTC AGC CGA TTT GCT ATC TCA TAC CAG GAG AAA GTC AAC CTC CTC TCT GCC GTC

K S P C P K D T P E G A E L K P W Y E P I Y L G G
AAG AGC CCC TGC CCC AAG GAC ACC CCT GAG GGG GCT GAG CTC AAA CCC TGG TAT GAG CCC ATA TAC CTG GGA GGA

V F Q L E K G D Q L S A E V N L P K Y L D F A E S
GTC TTC CAG CTG GAG AAG GGG GAC CAA CTC AGC GCT GAG GTC AAT CTG CCC AAG TAC TTA GAC TTT GCG GAG TCC

G Q V Y F G V I A L *
GGG CAG GTC TAC TTT GGA GTC ATT GCT CTG TGA GGA TCC

(aminoacid sequence: SEQ ID NO:24, nucleotide sequence: SEQ ID NO: 30)

### cDNA sequence cloned into the pET12 plasmid for murine NGR-TNF expression into the periplasmatic space of Escherichia coli cells.

Codons and the corresponding aminoacid (in bold above each codon) are indicated.

The restriction sites (*Sal I*) used for cDNA cloning into the pET12 plasmid are double underlined.
*, stop codon.
*Italics:* cDNA coding for CNGRCG sequence.
*Underlined:* cDNA sequence of murine TNF.
*Boxed sequence,* OmpT signal peptide (provided by the pET12 plasmid) to promote the export into the periplasmic space
*Arrow,* expected site of cleavage of the ompT-NGR-TNF fusion protein in *E. Coli.*

### Murine S-NGR-TNF cloned in pET/101D plasmid:

M S C N G R C G L R S S S Q N S S D K P V A H V
CACC ATG AGC TGC AAC GGC CGT TGC GGC CTC AGA TCA TCT TCT CAA AAT TCG AGT GAC AAG CCT GTA GCC CAC GTC

V A N H Q V E E Q L E W L S Q R A N A L L A N G M
GTA GCA AAC CAC CAA GTG GAG GAG CAG CTG GAG TGG CTG AGC CAG CGC GCC AAC GCC CTC CTG GCC AAC GGC ATG

D L K D N Q L V V P A D G L Y L V Y S Q V L F K G
GAT CTC AAA GAC AAC CAA CTA GTG GTG CCA GCC GAT GGG TTG TAC CTT GTC TAC TCC CAG GTT CTC TTC AAG GGA

Q G C P D Y V L L T H T V S R F A I S Y Q E K V N
CAA GGC TGC CCC GAC TAC GTG CTC CTC ACC CAC ACC GTC AGC CGA TTT GCT ATC TCA TAC CAG GAG AAA GTC AAC

L L S A V K S P C P K D T P E G A E L K P W Y E P
CTC CTC TCT GCC GTC AAG AGC CCC TGC CCC AAG GAC ACC CCT GAG GGG GCT GAG CTC AAA CCC TGG TAT GAG CCC

I Y L G G V F Q L E K G D Q L S A E V N L P K Y L
ATA TAC CTG GGA GGA GTC TTC CAG CTG GAG AAG GGG GAC CAA CTC AGC GCT GAG GTC AAT CTG CCC AAG TAC TTA

D F A E S G Q V Y F G V I A L *
GAC TTT GCG GAG TCC GGG CAG GTC TAC TTT GGA GTC ATT GCT CTG TGA GGA TCC

(amino acid sequence: SEQ ID NO:25, nucleotide sequence: 31)

### cDNA sequence cloned into the pET/101D plasmid for murine S-NGR-TNF expression in E.coli cells.

Chemically synthetized codons (optimized for expression in *Escherichia coli* cells) and the corresponding aminoacid (in bold above each codon) are indicated.

The restriction site (*Nde I and Bam HI*) used for cDNA cloning into the pET/101D plasmid are double underlined.
*, stop codon.

*Italics:* cDNA coding for SCNGRCG (SEQ ID NO:6) sequence.
*Underlined:* cDNA sequence of murine TNF.

### Human S-NGR-TNF cloned in pET/101D plasmid (codon usage optimized for Escherichia coli):

M S C N G R C G V R S S S R T P S D K P V A H V
CACC ATG AGC TGC AAC GGC CGT TGC GGC GTC AGA TCA TCT TCT CGA ACC CCG AGT GAC AAG CCT GTA GCC CAT GTT

V A N P Q A E G Q L Q W L N R R A N A L L A N G V
GTA GCA AAC CCT CAA GCT GAG GGG CAG CTC CAG TGG CTG AAC CGC CGG GCC AAT GCC CTC CTG GCC AAT GGC GTG

E L R D N Q L V V P S E G L Y L I Y S Q V L F K G
GAG CTG AGA GAT AAC CAG CTG GTG GTG CCA TCA GAG GGC CTG TAC CTC ATC TAC TCC CAG GTC CTC TTC AAG GGC

Q G C P S T H V L L T H T I S R I A V S Y Q T K V
CAA GGC TGC CCC TCC ACC CAT GTG CTC CTC ACC CAC ACC ATC AGC CGC ATC GCC GTC TCC TAC CAG ACC AAG GTC

N L L S A I K S P C Q R E T P E G A E A K P W Y E
AAC CTC CTC TCT GCC ATC AAG AGC CCC TGC CAG AGG GAG ACC CCA GAG GGG GCT GAG GCC AAG CCC TGG TAT GAG

P I Y L G G V F Q L E K G D R L S A E I N R P D Y
CCC ATC TAT CTG GGA GGG GTC TTC CAG CTG GAG AAG GGT GAC CGA CTC AGC GCT GAG ATC AAT CGG CCC GAC TAT

L D F A E S G Q V Y F G I I A L *
CTC GAC TTT GCC GAG TCT GGG CAG GTC TAC TTT GGG ATC ATT GCC CTG TGA GGA TCC

(amino acid sequence: SEQ ID NO:27, nucleotide sequence: 32)

### cDNA sequence cloned into the pET/101D plasmid for human S-NGR-TNF expression in E.coli cells.

Chemically synthetized codons (optimized for expression in *Escherichia coli* cells) and the corresponding aminoacid (in bold above each codon) are indicated.

The restriction site (*Nde I* and *Bam HI*) used for cDNA cloning into the pET/101D plasmid are double underlined.
*, stop codon.
*Italics:* cDNA coding for SCNGRCG (SEQ ID NO:6) sequence.

*Underlined:* cDNA sequence of human TNF.

### Human S-NGR-TNF cloned in pET11 plasmid:

M S C N G R C G V R S S S R T P S D K P V A H V
CAT ATG AGT TGC AAC GGC CGT TGC GGC GTC AGA TCA TCT TCT CGA ACC CCG AGT GAC AAG CCT GTA GCC CAT GTT

V A N P Q A E G Q L Q W L N R R A N A L L A N G V
GTA GCA AAC CCT CAA GCT GAG GGG CAG CTC CAG TGG CTG AAC CGC CGG GCC AAT GCC CTC CTG GCC AAT GGC GTG

E L R D N Q L V V P S E G L Y L I Y S Q V L F K G
GAG CTG AGA GAT AAC CAG CTG GTG GTG CCA TCA GAG GGC CTG TAC CTC ATC TAC TCC CAG GTC CTC TTC AAG GGC

Q G C P S T H V L L T H T I S R I A V S Y Q T K V
CAA GGC TGC CCC TCC ACC CAT GTG CTC CTC ACC CAC ACC ATC AGC CGC ATC GCC GTC TCC TAC CAG ACC AAG GTC

N L L S A I K S P C Q R E T P E G A E A K P W Y E
AAC CTC CTC TCT GCC ATC AAG AGC CCC TGC CAG AGG GAG ACC CCA GAG GGG GCT GAG GCC AAG CCC TGG TAT GAG

P I Y L G G V F Q L E K G D R L S A E I N R P D Y
CCC ATC TAT CTG GGA GGG GTC TTC CAG CTG GAG AAG GGT GAC CGA CTC AGC GCT GAG ATC AAT CGG CCC GAC TAT

L D F A E S G Q V Y F G I I A L *
CTC GAC TTT GCC GAG TCT GGG CAG GTC TAC TTT GGG ATC ATT GCC CTG TGA GGA TCC

(amino acid sequence: SEQ ID NO:27, nucleotide sequence: 33)

### cDNA sequence cloned into the pET11 plasmid for human S-NGR-TNF expression in E.coli cells.

Chemically synthetized codons and the corresponding aminoacid (in bold above each codon) are indicated.

The restriction sites (*Nde I* and *Sal I*) used for cDNA cloning into the pET11 plasmid are double underlined.
*, stop codon.
*Italics:* cDNA coding for SCNGRCG (SEQ ID NO:6) sequence.
*Underlined:* cDNA sequence of human TNF.

### Human NGR-TNF cloned in pPIC9K plasmid (codon usage optimized for Pichia pastoris):

M R F P S I F T A V L F A A S S A L A C N
GG ATC CAA ACG ATG AGA TTC CCT TCT ATT TTC ACT GCT GTT TTG TTC GCT GCT TCT TCT GCT TTG GCT TGT AAC

G R C G V R S S S R T P S D K P V A H V V A N P Q
GGT AGA TGT GGT GTT AGA TCT TCT TCT AGA ACT CCT TCT GAT AAG CCT GTT GCT CAT GTT GTT GCT AAC CCT CAA

A E G Q L Q W L N R R A N A L L A N G V E L R D N
GCT GAA GGT CAA TTG CAA TGG TTG AAC AGA AGA GCT AAT GCT TTG TTG GCT AAT GGT GTT GAA TTG AGA GAT AAT

Q L V V P S E G L Y L I Y S Q V L F K G Q G C P S
CAA TTG GTT GTT CCA TCT GAA GGT TTG TAC TTG ATC TAT TCT CAA GTT TTG TTC AAG GGT CAA GGT TGT CCT TCT

T H V L L T H T I S R I A V S Y Q T K V N L L S A
ACT CAT GTT TTG TTG ACT CAT ACT ATT TCT AGA ATC GCT GTT TCT TAT CAA ACT AAG GTT AAT TTG CTG TCT GCT

I K S P C Q R E T P E G A E A K P W Y E P I Y L G
ATT AAG TCT CCA TGT CAA AGA GAA ACT CCT GAA GGT GCT GAA GCT AAG CCA TGG TAC GAA CCA ATC TAT TTG GGT

G V F Q L E K G D R L S A E I N R P D Y L D F A E
GGT GTT TTT CAA TTG GAA AAG GGT GAC AGA TTG TCT GCT GAA ATT AAT AGA CCA GAT TAT TTG GAT TTC GCT GAA

S G Q V Y F G I I A L *
TCT GGT CAA GTT TAC TTT GGT ATT ATT GCT TTG TAA GAA TTC

(amino acid sequence: SEQ ID NO:29, nucleotide sequence: 34)

### cDNA sequence cloned into the pPIC9K plasmid for human NGR-TNF expression in Pichia pastoris cells.

Chemically synthetized codons (optimized for expression in *Pichia pastoris* cells) and the corresponding aminoacid (in bold above each codon) are indicated.

The restriction sites (*BamHI I* and *EcoRI*) used for cDNA cloning in the pPIC9K plasmid are double underlined.
*, stop codon.
*Boxed sequence,* alpha mating factor secretion signal peptide (provided by the pPIC9K expression plasmid) to promote the secretion of the NGR-TNF into the culture medium.
*Arrow,* site of cleavage of the fusion protein *in Pichia pastoris.*
*Italics:* cDNA coding for CNGRCG sequence.
*Underlined:* cDNA sequence of human TNF.

The present invention is therefore illustrated by means of non-limiting examples in reference to the following figures.
**Figure 1****. Schematic representation of the NGR deamidation reaction and of its products.** NGR transition to isoDGR and DGR in CNGRC (SEQ ID NO:5) peptides and conjugates can occur by nucleophilic attack of the backbone NH center on the Asn side-chain amide carbonyl, leading to loss of ammonia (-17 Da) and formation of a succinimide intermediate. Hydrolysis of succinimide leads to formation of isoDGR and DGR mixtures, with isoAsp and Asp in *L* and *D* configurations and gain of 1 Da.
**Figure 2****. MS analysis of human and murine CNGRCG-TNF.** Representative mass spectra of human and murine CNGRCG-TNF (NGR-TNF) or TNF as determined by ESI-MS using an API QStar PULSAR mass spectrometer (A) and Q Exactive HF mass spectrometer **(B-C).** Expected average masses are shown.

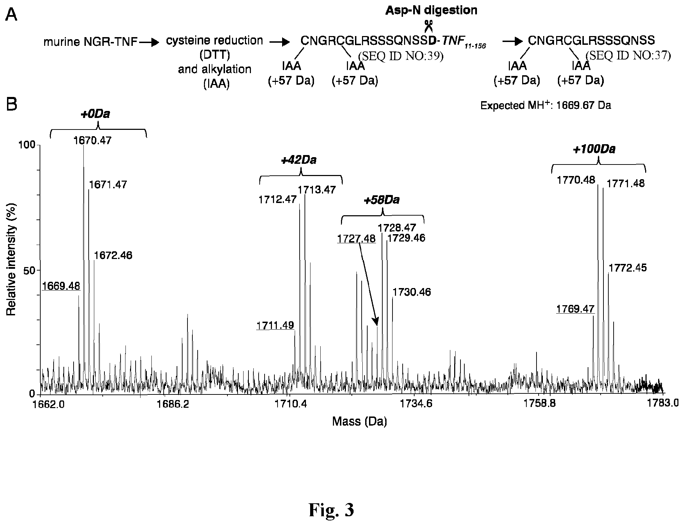

**Figure 3****. Digestion of murine NGR-TNF with Asp-N generates N-terminal fragments containing the +*****0Da**,* **+*****42Da**,* **+*58Da* and +*100Da* forms. A)** Schematic representation of sample preparation before mass spectrometry (MS) analysis. Murine NGR-TNF was reduced with 10 mM dithiothreitol (DTT), alkylated with 55 mM iodoacetamide (IAA) and digested with endoproteinase Asp-N for 16 h at 37°C in 0.1 M ammonium bicarbonate buffer, pH 8.0, containing 10% acetonitrile. **B**) Mass spectrum of the fragments corresponding to the N-terminal region of murine NGR-TNF, as detected by MALDI-TOF (Voyager-DE STR mass spectrometer, Applied Biosystems, Framingham, MA). The underlined numbers correspond to fragments with +0, +42, +58 and +100 Da, compared to the expected values for the unmodified N-terminal fragment of murine NGR-TNF. The other numbers correspond to isotopic distribution of each form.
**Figure 4****. MS analysis of different recombinant proteins expressed in *E.coli* cells.** Mass spectra of the indicated proteins as determined by ESI-MS using an API QStar PULSAR mass spectrometer. Expected average molecular masses are shown.
**Figure 5****. The auto-degradation product of murine NGR-TNF, lacking CNGRCG (SEQ ID NO:1), does not contain the +42Da, +58Da and +100Da molecular forms.**
   Mass spectrum of murine NGR-TNF after partial autoproteolysis (analyzed by ESI-MS, API QStar PULSAR mass spectrometer, PE-Sciex Instruments, Canada), showing an intact CNGRCG-TNF conjugate (heterogeneous) and a fragment corresponding to TNF alone (lacking CNGRCG (SEQ ID NO:1), homogeneous). Expected averages masses are shown.

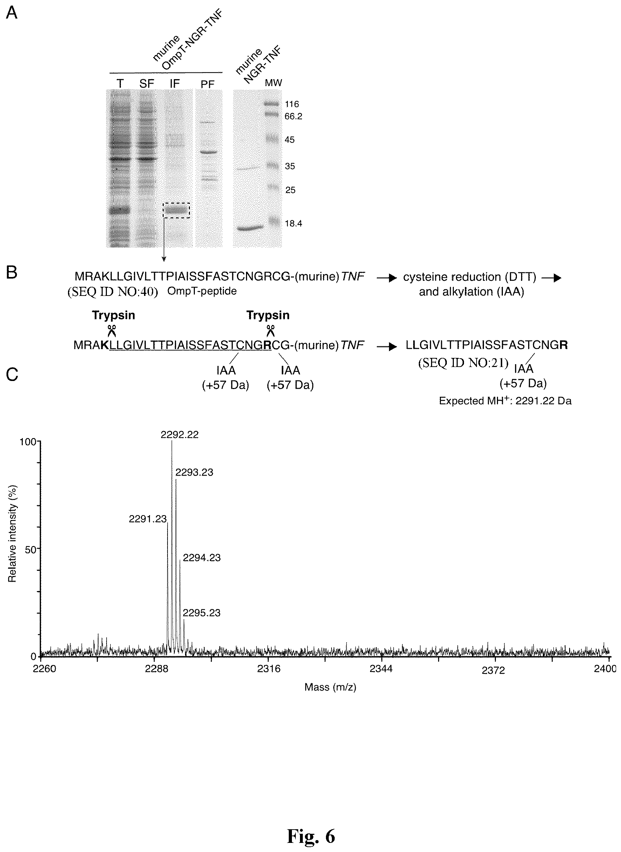

**Figure 6****. The OmpT-CNGRCG-TNF fusion protein does not contain the +42Da, +58Da and +100Da molecular forms. A)** SDS-PAGE analysis of protein extracts obtained from BL21 (DE3) E.coli cells transformed with the plasmid pET12 engineered to express murine CNGRCG-TNF. In this plasmid the cDNA coding for CNGRCG-TNF is fused to the C-terminus of the OmpT signal peptide (provided by the pET12 plasmid) to promote the export into the periplasmic space. Upon periplasmatic secretion the OmpT signal peptide is expected to be cleaved out from the fusion protein. *T,* total protein extract; *SF,* soluble protein fraction; *IF,* insoluble protein fraction; and *PF;* periplasmatic fraction. *MW,* molecular weight marker. Although no band corresponding to NGR-TNF is observed in the periplasmatic fraction, a band of a larger size of about 20 KDa is observed in the insoluble protein fraction (dashed rectangle, inclusion bodies). This suggests that the fusion protein was produced as insoluble inclusion bodies and that the OmpT sequence was not removed. **B)** Sample preparation workflow for mass spectrometry (MS) analysis. The gel containing the 20 KDa band (dashed rectangle, panel A) was excised and incubated with 10 mM dithiothreitol (DTT) in 50 mM ammonium bicarbonate pH 8.0) for 30 min at 56°C. The gel was then incubated with 55 mM iodoacetamide (IAA) in 50 mM ammonium bicarbonate) for 20 min at room temperature in the dark. After washing with water, the gel was incubated for 16 h at 37°C with a trypsin solution in 25 mM ammonium bicarbonate buffer pH 8.0, containing 5 mM CaCl₂. Finally, the peptides eluted from gel were analyzed by mass spectrometry using an MALDI-TOF Voyager-DE STR mass spectrometer (Applied Biosystems, Framingham, MA). C) Mass spectrum of the N-terminal region of OmpT-CNGRCG-TNF (murine) after reduction, alkylation and digestion with trypsin. The result shows that the signal peptide is not cleaved out from NGR-TNF and that the N-terminal fragment is homogeneous and without the +42, +58, and +100 Da modifications.

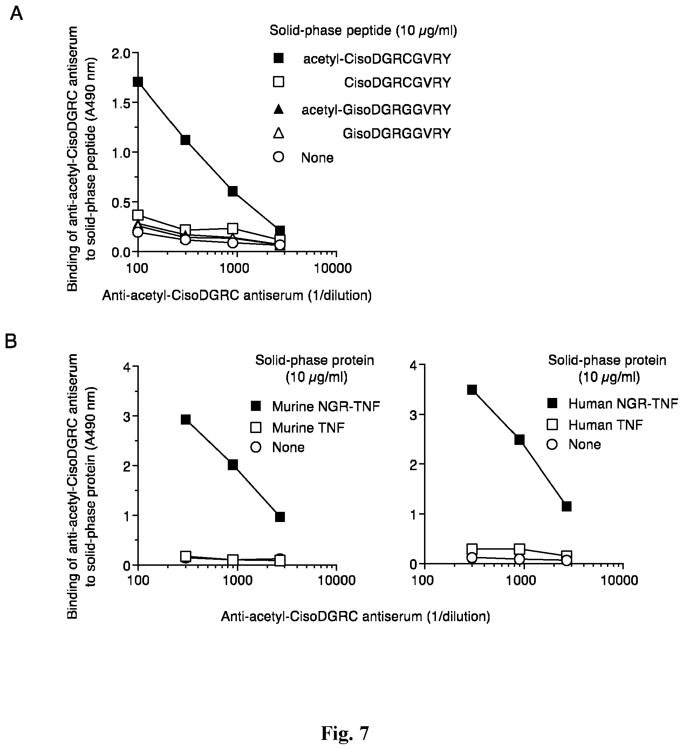

**Figure 7****. An anti-acetyl-CisoDGRC antiserum recognizes murine and human NGR-TNF, but not murine and human TNF.** An anti-acetyl-CisoDGRC (SEQ ID NO: 41) antiserum was obtained from rabbits immunized with the acetyl-CisoDGRCK (SEQ ID NO:36) peptide chemically coupled to ovalbumin via lysine ε-amino group. The capability of this antibody to recognize peptides and proteins bound to microtiter plates was then analysed by ELISA using a peroxidase-labeled goat-anti-rabbit antibody as a detecting reagent. **A**) Binding of anti-acetyl-CisoDGRC antibodies to microtiterplates coated with the indicated peptides containing isoDGR. The antiserum recognized the peptide containing the acetyl-CisoDGRC sequence, but not peptides containing the CisoDGRC or acetyl-GisoDGRC sequence. **B**) Binding of anti-acetyl-CisoDGRC to murine or human NGR-TNF and TNF. The antiserum recognized both murine and human deamidated NGR-TNF, but not to TNF, suggesting that both products contained acetyl-cysteine groups.

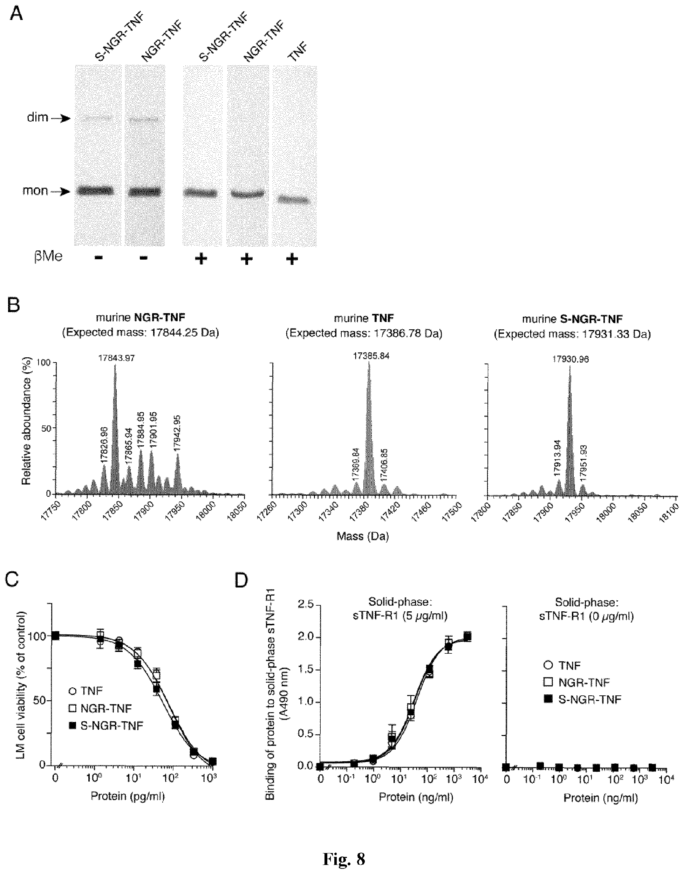

**Figure 8****. Biochemical and biological characterization of murine S-NGR-TNF and NGR-TNF. A**) SDS-PAGE under reducing (βMe +) and non-reducing conditions (βMe -) of murine S-NGR-TNF, NGR-TNF and TNF. *Arrows:* bands corresponding to monomeric subunits (*mon*) and reducible dimers *(dim).* **B)** Representative mass spectra of murine NGR-TNF, TNF and S-NGR-TNF as determined by a Q Exactive HF mass spectrometer. Expected average masses are shown. **C**) Cytotoxic activity of TNF, NGR-TNF and S-NGR-TNF against L-M cells. L-M cells were incubated in DMEM medium supplemented with 2 mM glutamine, 100 U/ml penicillin, 100 µg/ml streptomycin, 0.25 µg/ml amphotericin-B, 10% fetal bovine serum, 2 µg/ml actinomycin D and TNF, NGR-TNF or S-NGR-TNF at the indicated doses (20 h at 37°C, 5% CO₂). Cell viability was quantified by standard 3-(4,5-dimethylthiazol-2-yl)-2,5-diphenyltetrazolium bromide (MTT) assay. **D**) Binding of TNF, NGR-TNF and S-NGR-TNF to sTNF-R1 (Etanercept). Microtiterplates were precoated with or without the indicated amount of sTNF-R1 (for 16 h), washed with PBS, blocked with PBS containing 3 % milk, 1 % BSA (binding buffer, 1 h) and incubated with the indicated amounts of TNF, NGR-TNF or S-NGR-TNF diluted in binding buffer (for 1.5 h). The binding of each protein to sTNF-R1 was then detected using a polyclonal anti-murine TNF (IP301, 5 µg/ml in binding buffer, 1 h) followed by a polyclonal goat anti-rabbit antibody conjugated with horseradish peroxidase (1:1000 in binding buffer, 1 h) and o-phenylendiammine as chromogenic substrate.

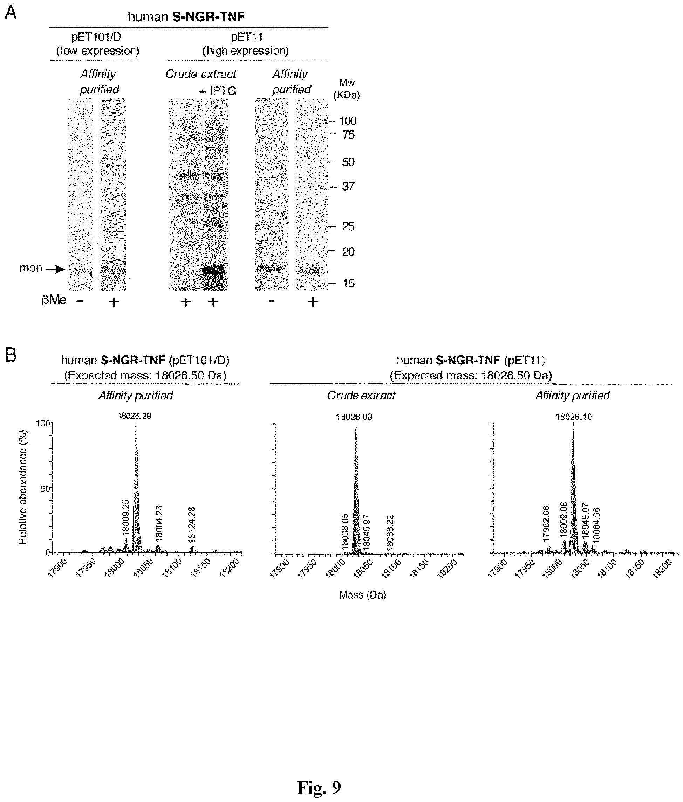

**Figure 9****. Characterization of human S-NGR-TNF and NGR-TNF produced with different expression systems in E. coli cells. A)** SDS-PAGE under reducing (βMe +) and non-reducing conditions (βMe -) of human S-NGR-TNF expressed in *E.coli* cells using the pET101/D and pET11 plasmid, which allow low and high expression levels. *Arrows:* bands corresponding to monomeric subunits (*mon*). **B**) Representative mass spectra of human S-NGR-TNF (crude extracts and affinity-purified), as determined by a Q Exactive HF mass spectrometer. Expected average masses are shown. Human S-NGR-TNF was purified from *E.coli* cell extract by affinity chromatography on sTNF-R1 (etanercept)-agarose column and analyzed by mass spectrometry without further additional purification steps.

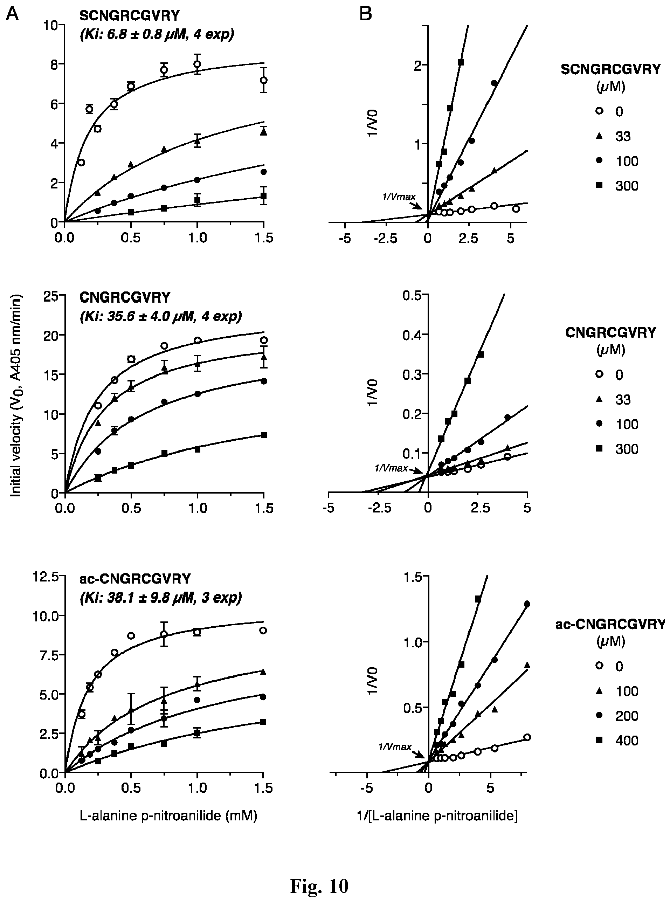

**Figure 10****. The peptide SCNGRCGVRY (SEQ ID NO: 3) inhibits CD13 enzymatic activity more efficiently than CNGRCGVRY (SEQ ID NO:4) and acetylated-CNGRCGVRY. A**) Steady state kinetic analysis of CD13 in the presence of various amounts of L-alanine p-nitroanilide substrate and SCNGRCGVRY (SEQ ID NO: 3), CNGRCGVRY (SEQ ID NO:4), or acetylated-CNGRCGVRY (ac-CNGRCGVRY) (SEQ ID NO:42). A representative plot of 3-4 independent experiments, showing the initial velocity (V₀) versus substrate concentration (mean ± SE of four technical replicates), is shown. The assays were performed with recombinant human histidine-tagged CD13 (200-300 ng/ml) in 60 mM potassium phosphate buffer, pH 7.4, at room temperature. The kinetic of p-nitroanilide formation was monitored spectrophotometrically (A405 nm) for 5-10 min. The inhibitory constant (*Ki*) reported in each plot is the result of the 3-4 independent experiments (mean ± SE). **B**) Double reciprocal plots of the experiments reported in panel A.

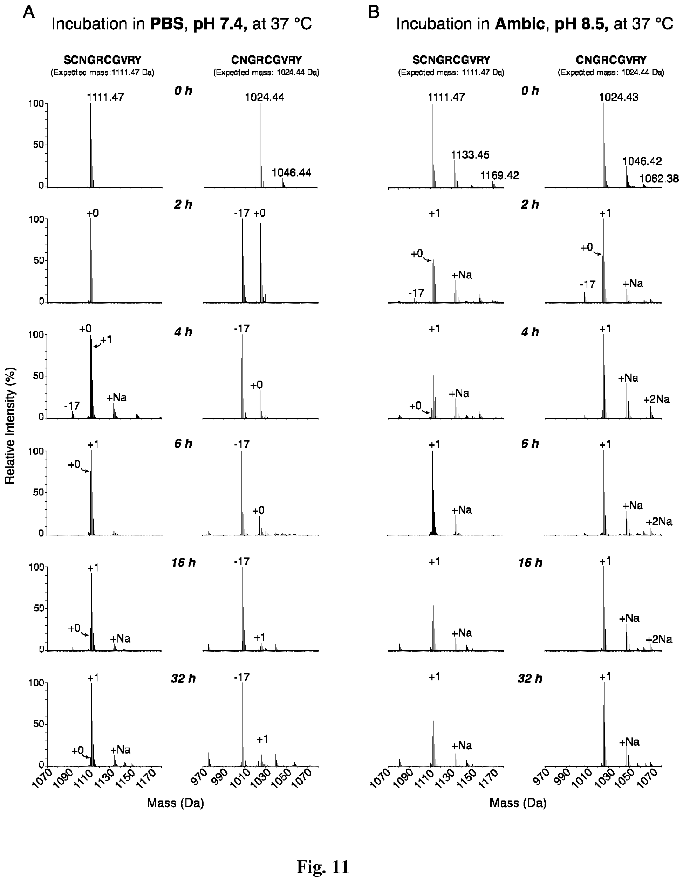

**Figure 11****. The peptide SCNGRCGVRY (SEQ ID NO: 3) has a lower propensity to deamidate than CNGRCGVRY (SEQ ID NO:4) in a physiological buffer at pH7.3, but not in ammonium bicarbonate buffer at pH 8.5. A** and **B**) MS analysis of SCNGRCGVRY (SEQ ID NO: 3) and CNGRCGVRY (SEQ ID NO:4) after incubation at 37 °C for the indicated time in 50 mM sodium phosphate buffer, pH 7.4 (PBS) or 0.1 M ammonium bicarbonate buffer, pH 8.5 (Ambic). The values *+0,* + *1*, *-17* reported in the plot correspond to the difference between the observed and the expected molecular mass of peptides expressed in daltons. MS analysis was performed using the LTQ-Orbitrap mass spectrometer (Thermo Scientific).

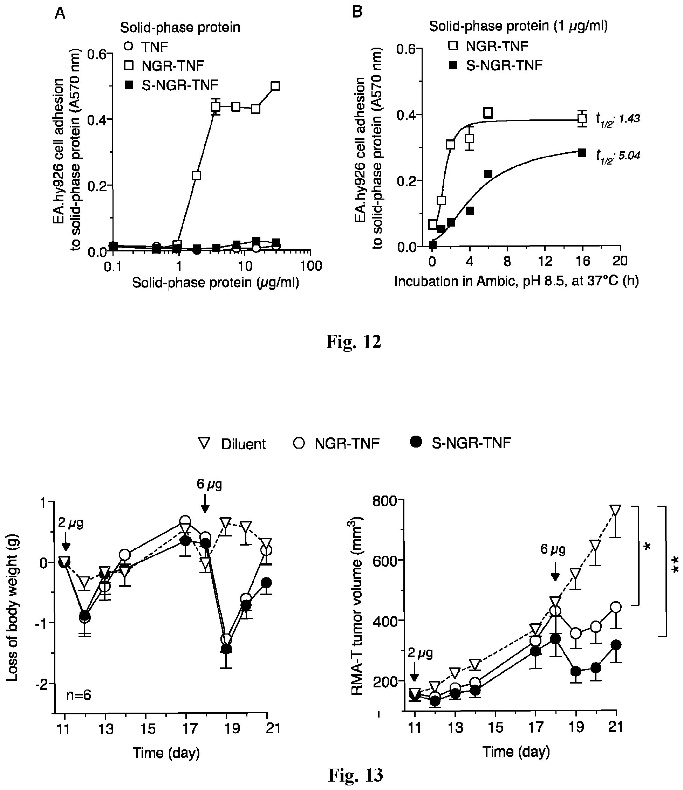

**Figure 12****. S-NGR-TNF does not promote endothelial EA.hy926 cell adhesion, unless treated with 0.1 M ammonium bicarbonate buffer, pH 8.5, to force deamidation. A**) Adhesion of endothelial EA.hy926 cells to 96-wells microtiterplates coated with different amounts of murine TNF, NGR-TNF and S-NGR-TNF in 150 mM sodium chloride, 50 mM sodium phosphate buffer, pH 7.4 (PBS) (16 h). Each well was blocked with 2% BSA in PBS containing calcium chloride and magnesium chloride (DPBS, 30 min), seeded with 40.000 endothelial EA.hy926 cells in DPBS supplemented with 0.1% BSA (DPBS-B), and left to incubate for 2 h at 37 °C, 5% CO₂. After washing with DMEM-B, adherent cells were stained with crystal violet and quantified by spectrophotometric measurements at 570 nm. **B**) Microtiterplates pre-coated with 1 µg/ml of murine NGR-TNF or S-NGR-TNF in PBS (for 16 h) were incubated 0.1 M ammonium bicarbonate buffer, pH 8.5 (Ambic) at 37°C for the indicated time to promote NGR deamidation. EA.hy926 cell adhesion assay was then performed as described above.
**Figure 13****. The addition of a serine residue to the N-terminus of NGR-TNF does not impair its anti-tumor activity and does not increase its toxicity at high doses. A**) Effect of high-dose murine S-NGR-TNF or NGR-TNF on the body weight and tumor volume of RMA-T lymphoma- bearing mice. Six C57BL/6 mice (6-7 weeks old, weighing 18-20 g) were challenged with subcutaneous injection in the left flank of 5×10⁴ RMA-T cells. Mice bearing RMA lymphomas (6 per group) were treated at day 11 after tumor implantation with 2 µg and 6 µg of murine NGR-TNF or S-NGR-TNF in 0.9% sodium chloride, containing 100 µg/ml endotoxin-free human albumin (i.p.) as indicated (arrows). Animal weights and tumor growth were monitored as indicated. **B**) Effect of melphalan alone or in combination with low-dose murine S-NGR-TNF on the body weight of RMA-T lymphoma-bearing mice. Six tumor-bearing mice were injected with the indicated doses of S-NGR-TNF (i.p) and, two hours later, with the generic preparation of melphalan (Melphalan-Tillomed) (i.p.). *, P<0.05, **P<0.01, by *unpaired t test* analysis of the area under the curve for each tumor using the GraphPad Prism software.

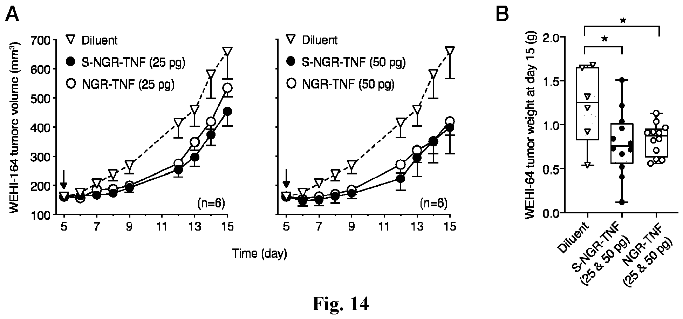

**Figure 14****. The addition of a serine residue to the N-terminus of NGR-TNF does not impair its anti-tumor activity and does not increase its toxicity at low doses.** Balb/c mice (6-7 weeks old, weighing 18-20 g) were injected, s.c., with 1.5×10⁶ WEHI-164 cells in the left flank and treated 5 days after tumor implantation (i.p.) with 25 or 50 pg of murine NGR-TNF or S-NGR-TNF in 0.9% sodium chloride, containing 100 µg/ml endotoxin-free human serum albumin (arrows). **A**) Tumor volumes after treatment (mean±SE, 6 mice per group). **B**) Tumor weight at day 15 (cumulative data of the two groups treated with 25 and 50 pg of drugs). *, P<0.05, **P<0.01 by *unpaired t test* of the area under the curve for each tumor (GraphPad Prism software).

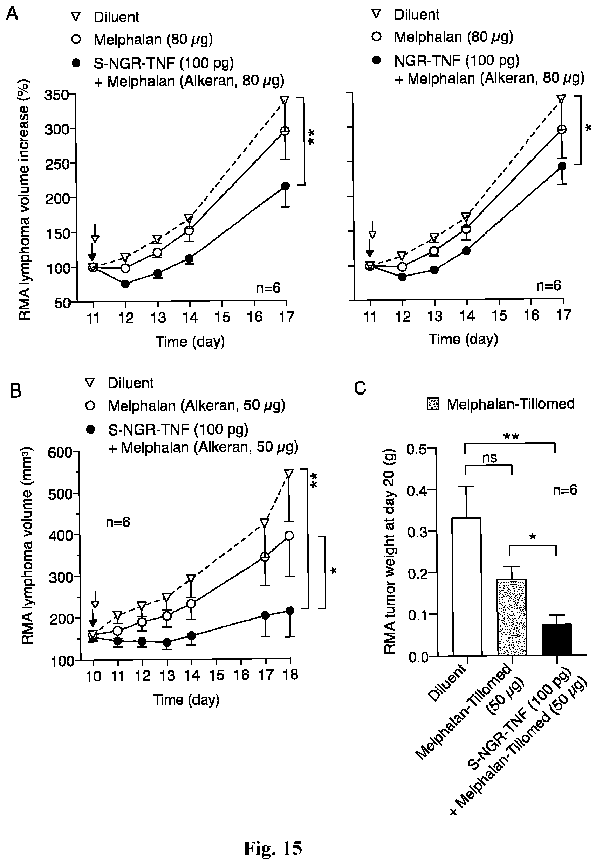

**Figure 15****. The addition of a serine residue to the N-terminus of NGR-TNF does not impair its anti-tumor activity in combination with melphalan.** C57BL/6 mice (6-7 weeks old, weighing 18-20 g) were challenged with subcutaneous injection in the left flank of 7×10⁴ RMA cells. Mice bearing RMA lymphomas (6 per group) were treated 10-11 days (after tumor implantation with 100 pg of murine NGR-TNF or S-NGR-TNF (i.p.). Two hours later, the mice were injected with the indicated dose of melphalan (i.p.). All proteins were diluted with 0.9% sodium chloride, containing 100 µg/ml endotoxin-free human albumin. Tumor growth was monitored daily by measuring the tumors with calipers. **A-B**) Tumor volume change of two independent experiments are shown (mean±SE). These experiments were carried out using melphalan (Alkeran) from Aspen Pharma. *, P<0.05, **P<0.01 by *unpaired t test* analysis of the area under the curve for each tumor, with the GraphPad Prism software. **C**) Tumor bearing-animals were treated at day 10 after tumor implantation as indicated above using the indicated dose of S-NGR-TNF and the generic preparation of melphalan (Melphalan-Tillomed, from Tillomed Italia). RMA tumor weight at day 20 are shown (mean±SE). *, P<0.05, **P<0.01 by *unpaired t test* analysis with the GraphPad Prism software.

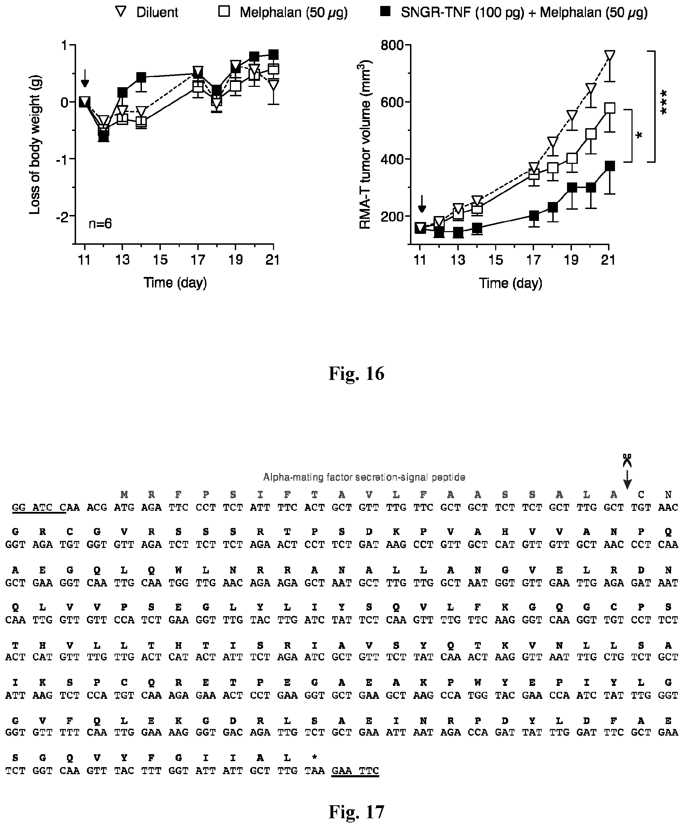

**Figure 16****. The addition of a serine residue to the N-terminus of NGR-TNF does not exacerbate the toxicity of melphalan**
   Effect of melphalan alone or in combination with low-dose murine S-NGR-TNF on the body weight and tumor growth of RMA-T lymphoma-bearing mice. Six tumor-bearing mice were injected with the indicated doses of S-NGR-TNF (i.p) and, two hours later, with the generic preparation of melphalan (Melphalan-Tillomed) (i.p.). Tumor weight and tumor volumes were monitored dayly. *, P<0.05, ***P<0.001, by *unpaired t test* analysis of the area under the curve for each tumor using the GraphPad Prism software.
**Figure 17****. cDNA sequence cloned into the pPIC9K plasmid for NGR-hTNF expression in Pichia pastoris cells.**
   Chemically synthetized codons and the corresponding aminoacid (in bold above each codon) are indicated. The restriction sites (*BamHI* and *EcorRI*) used for cDNA cloning into the pPIC9K plasmid are underlined. *, stop codon. *Arrow,* site of cleavage of the fusion protein in *Pichia pastoris.*

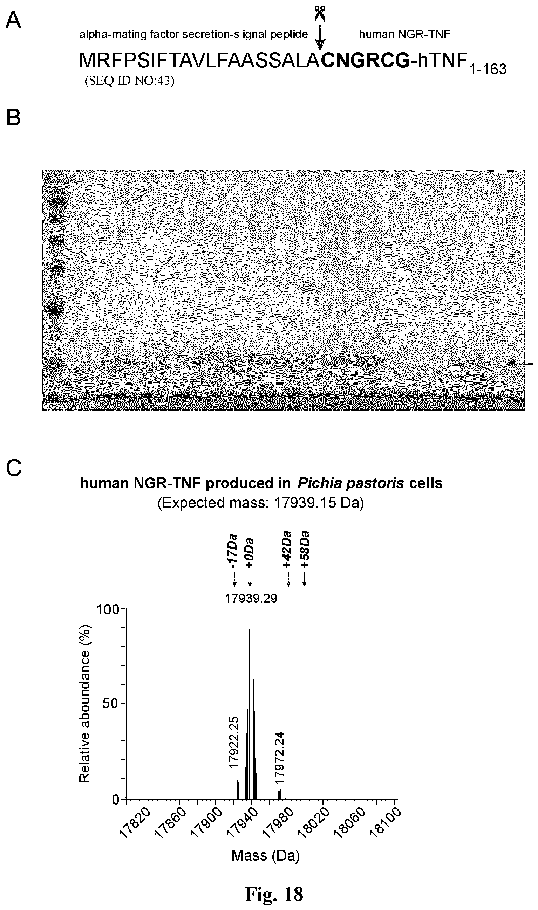

**Figure 18****. Characterization of human CNGRCG-TNF (NGR-TNF) expressed in *Pichia pastoris* cells.**
   *Pichia pastoris* cells (strain *GS115 and K)* were engineered to express human CNGRCG-TNF. In this expression system the cDNA coding for CNGRCG-TNF is fused to the C-terminus of the *alpha mating factor* secretion signal peptide (provided by the pPIC9K expression plasmid) to promote the secretion into the culture medium. **A**) Sequence of the fusion protein and the expected site of cleavage (arrow). **B**) SDS-PAGE analysis of the culture medium obtained from wild type Pichia pastoris cells (∅) or Pichia pastoris clones engineered to express human NGR-TNF. Cell cultures were induced at 28°C by adding methanol (1%) for 48 h. MW, molecular weight marker is shown. Clone #1, #2 and #4 of strain K cells do not express NGR-TNF, whereas a band of about 17 KDa was observed with all the other clones. C) Human NGR-TNF was purified from cell culture medium by affinity chromatography on sTNF-R1 (etanercept)-agarose column and analyzed by mass spectrometry using an ESI-MS Q Exactive HT mass spectrometer. Mass spectrum of NGR-TNF and expected average mass are shown.

When describing the present invention, all terms not defined herein have their common art-recognized meanings. Any term or expression not expressly defined herein shall have its commonly accepted definition understood by those skilled in the art. To the extern that the following description is of a specific embodiment or a particular use of the invention, it is intended to be illustrative only, and not limiting of the claimed invention. The following description is intended to cover all alternatives, modifications and equivalents that are included in the spirit and scope of the invention, as defined in the appended claims.

The present invention also includes functional fragments, variants or derivatives of the proteins, peptides, conjugates or sequences herein disclosed. In the context of the present invention, when referring to specific DNA sequences, it is intended that it is comprised within the invention also RNA molecules identical to said polynucleotides, except for the fact that the RNA sequence contains uracil instead of thymine and the backbone of the RNA molecule contains ribose instead of deoxyribose, RNA sequence complementary the sequences therein disclosed, functional fragments, mutants and derivatives thereof, proteins encoded therefrom, functional fragments, mutants and derivatives thereof. The term "complementary" sequence refers to a polynucleotide which is non-identical to the sequence but either has a complementary base sequence to the first sequence or encodes the same amino acid sequence as the first sequence. A complementary sequence may include DNA and RNA polynucleotides. The term "functional" or "functional" may be understood as capable of maintaining the same activity. "Fragments" are preferably long at least 10 aa., 20 aa., 30 aa., 40 aa., 50 aa., 60 aa., 70 aa., 80 aa., 90 aa., 100 aa., ... "Derivatives" may be recombinant or synthetic. The term "derivative" as used herein in relation to a protein means a chemically modified protein or an analogue thereof, wherein at least one substituent is not present in the unmodified protein or an analogue thereof, i.e. a protein which has been covalently modified. Typical modifications are amides, carbohydrates, alkyl groups, acyl groups, esters and the like. As used herein, the term "derivatives" also refers to longer or shorter polynucleotides/proteins and/or having e.g. a percentage of identity of at least 85%, 86%, 87%, 88%, 89%, 90%, 91%, 92%, 93%, 94%, 95%, 96%, 97%, 98%, more preferably of at least 99% with the sequences herein disclosed. In the present invention "at least 70 % identity" means that the identity may be at least 70%, or 75%, or 80%, or 85 % or 90% or 95% or 100% sequence identity to referred sequences. This applies to all the mentioned % of identity. Preferably, the % of identity relates to the full length of the referred sequence. The derivative of the invention also includes "functional mutants" of the polypeptides, which are polypeptides that may be generated by mutating one or more amino acids in their sequences and that maintain their activity. Indeed, the polypeptide of the invention, if required, can be modified in vitro and/or in vivo, for example by glycosylation, myristoylation, amidation, carboxylation or phosphorylation, and may be obtained, for example, by synthetic or recombinant techniques known in the art. In the present invention "functional" is intended for example as "maintaining their activity" e.g. immunomodulatory activity or anti-inflammatory activity. Also within the scope of the subject invention are polynucleotides which have the same nucleotide sequences of a polynucleotide exemplified herein except for nucleotide substitutions, additions, or deletions within the sequence of the polynucleotide, as long as these variant polynucleotides retain substantially the same relevant functional activity as the polynucleotides specifically exemplified herein (e.g., they encode a protein having the same amino acid sequence or the same functional activity as encoded by the exemplified polynucleotide). Thus, the polynucleotides disclosed herein should be understood to include mutants, derivatives, variants and fragments, as discussed above, of the specifically exemplified sequences. The subject invention also contemplates those polynucleotide molecules having sequences which are sufficiently homologous with the polynucleotide sequences of the invention so as to permit hybridization with that sequence under standard stringent conditions and standard methods (Maniatis, T. et al, 1982). Polynucleotides described herein can also be defined in terms of more particular identity and/or similarity ranges with those exemplified herein. The sequence identity will typically be greater than 60%, preferably greater than 75%, more preferably greater than 80%, even more preferably greater than 90%, and can be greater than 95%. The identity and/or similarity of a sequence can be 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91 , 92, 93, 94, 95, 96, 97, 98, or 99% or greater as compared to a sequence exemplified herein. Unless otherwise specified, as used herein percent sequence identity and/or similarity of two sequences can be determined using the algorithm of Karlin and Altschul (1990), modified as in Karlin and Altschul (1993). Such an algorithm is incorporated into the NBLAST and XBLAST programs of Altschul et al. (1990). BLAST searches can be performed with the NBLAST program, score = 100, wordlength = 12, to obtain sequences with the desired percent sequence identity. To obtain gapped alignments for comparison purposes, Gapped BLAST can be used as described in Altschul et al. (1997). When utilizing BLAST and Gapped BLAST programs, the default parameters of the respective programs (NBLAST and XBLAST) can be used. See NCBI/NIH website.

The peptide, the protein or the compound X as above defined may include an amino acid sequence with at least 65%, 70%, 75%, 80%, 82 %, 85 %, 90 %, 92 %, 95%, 98%, 99% or 100% identity to SEQ ID NO: 1 or with other sequences herein mentioned. Determining percent identity of two amino acid sequences may include aligning and comparing the amino acid residues at corresponding positions in the two sequences. If all positions in two sequences are occupied by identical amino acid residues then the sequences are said to be 100% identical. Percent identity may be measured by the Smith Waterman algorithm (Smith T F, Waterman M S 1981 "Identification of Common Molecular Subsequences," J Mol Biol 147: 195-197, which is incorporated herein by reference as if fully set forth). The peptide, the protein or the compound X may have fewer or more than the residues of the mentioned sequences. E.g. the peptide may include more than 6 amino acids. The peptide, the protein or the compound X may present amino acid replacement in comparison to the sequence of SEQ ID NO. 1 or to the other herein mentioned sequences. The replacement may be with any amino acid whether naturally occurring or synthetic. The replacement may be with an amino acid analogue or amino acid mimetic that functions similarly to the naturally occurring amino acids. Naturally occurring amino acids are those encoded by the genetic code, as well as those amino acids that are later modified. The later modification may be but is not limited to hydroxyproline, γ-carboxyglutamate, and O-phosphoserine modifications. Naturally occurring amino acids include the standard 20, and unusual amino acids. Unusual amino acids include selenocysteine. The replacement may be with an amino acid analogue, which refers to compounds that have the same basic chemical structure as a naturally occurring amino acid; e.g., a carbon that is bound to a hydrogen, a carboxyl group, an amino group, and an R group. Examples of amino acid analogues include but are not limited to homoserine, norleucine, methionine sulfoxide, methionine methyl sulfonium. Such analogues may have modified R groups or modified peptide backbones. The amino acid analogues may retain the same basic chemical structure as a naturally occurring amino acid. The replacement may be with an aminoacid mimetic, which refers to chemical compounds that have a structure that is different from the general chemical structure of an amino acid, but that functions similarly to a naturally occurring amino acid. The replacement may be with an α,α-disubstituted 5-carbon olefinic unnatural amino acid. A replacement may be a conservative replacement, or a non-conservative replacement. A conservative replacement refers to a substitution of an amino acid with a chemically similar amino acid. Conservative substitution tables providing functionally similar amino acids are well known in the art. Such conservatively replacements include but are not limited to substitutions for one another: (1) Alanine (A), Glycine (G); (2) Aspartic acid (D), Glutamic acid (E); (3) Asparagine (N), Glutamine (Q); (4) Arginine (R), Lysine (K); (5) Isoleucine (I), Leucine (L), Methionine (M), Valine (V); (6) Phenylalanine (F), Tyrosine (Y), Tryptophan (W); (7) Serine (S), Threonine (T); and (8) Cysteine (C), Methionine (M) (see, e.g., Creighton, Proteins (1984)). A replacement may be from one amino acid to another with a similar hydrophobicity, hydrophilicity, solubility, polarity, or acidity. A sequence having less than 100% identity to the reference sequence SEQ ID NO:1 or to other mentioned sequences may be referred to as a variant. An embodiment includes a composition including the peptide having a sequence that is a variant of SEQ ID NO: 1. In an embodiment, one or more amino acids residues are replaced with a residue having a crosslinking moiety. As used herein, a "peptide" or "polypeptide" comprises a polymer of amino acid residues linked together by peptide (amide) bonds. The term(s), as used herein, refer to proteins, polypeptides, and peptide of any size, structure, or function. Typically, a peptide or polypeptide will be at least three amino acids long. A peptide or polypeptide may refer to an individual protein or a collection of proteins. The peptides of the instant invention may contain natural amino acids and/or non-natural amino acids (i.e., compounds that do not occur in nature but that can be incorporated into a polypeptide chain). Amino acid analogues as are known in the art may alternatively be employed. One or more of the amino acids in a peptide or polypeptide may be modified, for example, by the addition of a chemical entity such as a carbohydrate group, a hydroxyl group, a phosphate group, a farnesyl group, an isofarnesyl group, a fatty acid group, a linker for conjugation, functionalization, or other modification. A peptide or polypeptide may also be a single molecule or may be a multi-molecular complex, such as a protein. A peptide or polypeptide may be just a fragment of a naturally occurring protein or peptide. A peptide or polypeptide may be naturally occurring, recombinant, or synthetic, or any combination thereof. A large number of agents are developed to target cellular contents, cellular compartments, or specific protein, lipid, nucleic acid or other targets or biomarkers within cells. While these agents can bind to their intracellular targets with strong affinity, many of these compounds, whether they be molecules, proteins, nucleic acids, peptides, nanoparticles, or other intended therapeutic agents or diagnostic markers cannot cross the cell membrane efficiently or at all.

The composition may include a pharmaceutically acceptable carrier. The pharmaceutically acceptable carrier may include but is not limited to at least one of ion exchangers, alumina, aluminum stearate, lecithin, serum proteins, human serum albumin, buffer substances, phosphates, glycine, sorbic acid, potassium sorbate, partial glyceride mixtures of saturated vegetable fatty acids, water, salts, electrolytes, protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, waxes, polyethylene glycol, starch, lactose, dicalcium phosphate, microcrystalline cellulose, sucrose, dextrose, talc, magnesium carbonate, kaolin; non-ionic surfactants, edible oils, physiological saline, bacteriostatic water, Cremophor EL™ (BASF, Parsippany, N.J.), and phosphate buffered saline (PBS).

Administering may include delivering a dose of 1 ng/kg/day to 100 µg/kg/day of the fusion protein or conjugate product. The dose may be any value between 1 ng/kg/day to 100 µg/kg/day. The dose may be any dose between and including any two integer values between 1 ng/kg/day to 100 µg/kg/day. The dose may be 10, 15, 20, 25, 30, 35, 40, 45, 50, 55, 60, 65, 70, 75, 80, 85, 90, 95, or 100 ng/kg/day or mg/kg/day or any dose in a range between any two of the foregoing. Preferably, the dose may be about 16 ng/kg/day. Administering may include delivering any dose of a complementing therapeutic. The complementing therapeutic dose may be any 1 to 100 mg/kg/day. The complementing therapeutic dose may be any value between 1 to 100 mg/kg/day. The complementing therapeutic dose may be any dose between and including any two integer values between 1 ng/kg/day to 100 mg/kg/day. The complementing therapeutic dose may be 10, 15, 20, 25, 30, 35, 40, 45, 50, 55, 60, 65, 70, 75, 80, 85, 90, 95, or 100 mg/kg/day or any dose in a range between any two of the foregoing. The complementing therapeutic may be any one or more of nanoparticle (e.g. gold nanoparticles, liposomes), a therapeutic agent (e.g. cytokines, chemotherapeutic drugs, antibodies and antibody fragments, toxins, nucleic acids), a diagnostic agent (e.g. radioactive compounds, fluorescence compounds, chemiluminescent compounds), a contrasting agent (e.g. microbubbles), or cellular components (e.g. chimeric antigen receptors or TCRs). The concentration of the peptide(s) and at least one complementing therapeutic in the composition may be set to deliver the daily (or weekly, every three weeks or monthly) dosage in a single administration, two-point administrations, multiple point administrations, or continuous administration (e.g., intravenously, transdermally, intraperitoneally, by isolated limb perfusion, by isolated hepatic perfusion, or local administration) over a period of time. The period may be one day. The period may be 1, 2, 4, 8, 12, or 24 hours or a time within a range between any two of these values. The peptide-cytokine and complementing therapeutic can be administered simultaneously or with 1, 2, 4, 8, 12, 24, 48 hours of delay or anticipation or any intermediate times.

A composition including fusion protein or conjugate product of the invention may include any amount of the protein or product. The amount may be that sufficient to deliver the dosage as set forth above in a suitable volume or sized delivery mode. When the dosage is split into multiple administrations throughout a time period, the amount in one volume or delivery mode may be the total dosage divided by the number of administrations throughout the time period. When present in a composition, the complementing therapeutic may be at any complementing therapeutic amount. Like the peptide, the complementing therapeutic amount may be tailored to deliver the right complementing therapeutic amount in the volume or delivery mode used for administration.

The patient may be an animal. The patient may be a mammal. The patient may be a human. The patient may be a cancer patient. The cancer patient may be a lymphoma, or sarcoma, melanoma oral or skin squamous cell carcinoma, hepatocellular carcinoma, head and neck, gastroesophageal, colorectal, pancreatic, ovarian, lung, cervix, breast cancer, renal, urothelial, brain tumors (e.g. glioblastoma and astrocytoma) cancer patient, or patients with other solid-tumors or with metastasis of said tumors. The route for administering a composition or pharmaceutical composition may be by any route. The route of administration may be any one or more route including but not limited to oral, injection, topical, enteral, rectal, gastrointestinal, sublingual, sublabial, buccal, epidural, intracerebral, intracerebroventricular, intracisternal, epicutaneous, intraderm al, subcutaneous, nasal, intravenous, intraarterial, intramuscular, intracardiac, intraosseous, intrathecal, intraperitoneal, intravesical, intravitreal, intracavernous, intravaginal, intrauterine, extra-amniotic, transdermal, intratumoral, and transmucosal. Embodiments include a method of making the peptides of the invention, including the stapled peptide.

The method may include synthesizing a fusion protein or conjugate product having the sequence of the selected modified peptide.

The method may include evaluating the binding to CD13 of the peptide. Methods and conditions for evaluating the binding of the peptide may be set forth in the Example below.

An embodiment includes fusion protein or conjugate product or a composition thereof comprising a peptide consisting of, consisting essentially of, or comprising the sequence of any amino acid sequence herein. The peptide composition may include any complementing therapeutic herein. The peptide composition may include a pharmaceutically acceptable carrier.

The term "protein" includes single-chain polypeptide molecules as well as multiple-polypeptide complexes where individual constituent polypeptides are linked by covalent or non-covalent means. The term "polypeptide" includes peptides of two or more amino acids in length, typically having more than 5, 10 or 20 amino acids.

It will be understood that polypeptide sequences for use in the invention are not limited to the particular sequences or fragments thereof but also include homologous sequences obtained from any source, for example related viral bacterial proteins, cellular homologues and synthetic peptides, as well as variants or derivatives thereof. Polypeptide sequences of the present invention also include polypeptides encoded by polynucleotides of the present invention.

The terms "variant" or "derivative" in relation to the amino acid sequences of the present invention includes any substitution of, variation of, modification of, replacement of, deletion of or addition of one (or more) amino acids from or to the sequence providing the resultant amino acid sequence preferably has targeting activity, preferably having at least 25 to 50% of the activity as the polypeptides herein presented, more preferably at least substantially the same activity. Thus, sequences may be modified for use in the present invention. Typically, modifications are made that maintain the activity of the sequence. Thus, in one embodiment, amino acid substitutions may be made, for example from 1, 2 or 3 to 10, 20 or 30 substitutions provided that the modified sequence retains at least about 25 to 50% of, or substantially the same activity. However, in an alternative embodiment, modifications to the amino acid sequences of a polypeptide of the invention may be made intentionally to reduce the biological activity of the polypeptide. For example, truncated polypeptides that remain capable of binding to target molecule but lack functional effector domains may be useful. In general, preferably less than 20%, 10% or 5% of the amino acid residues of a variant or derivative are altered as compared with the corresponding region depicted in the sequence listings. Amino acid substitutions may include the use of non-naturally occurring analogues, for example to increase blood plasma half-life of a therapeutically administered polypeptide.

Polypeptides of the invention also include fragments of the above-mentioned polypeptides and variants thereof, including fragments of the sequences. Preferred fragments include those which include an epitope. Suitable fragments will be at least about 5, e.g. 10, 12, 15 or 20 amino acids in length. They may also be less than 200, 100 or 50 amino acids in length. Polypeptide fragments of the proteins and allelic and species variants thereof may contain one or more (e.g. 2, 3, 5, or 10) substitutions, deletions or insertions, including conserved substitutions. Where substitutions, deletion and/or insertions have been made, for example by means of recombinant technology, preferably less than 20%, 10% or 5% of the amino acid residues depicted in the sequence listings are altered. Proteins of the invention are typically made by recombinant means. However, they may also be made by synthetic means using techniques well known to skilled persons such as solid phase synthesis. Various techniques for chemical synthesising peptides are reviewed by Borgia and Fields, 2000, TibTech 18: 243-251 and described in detail in the references contained therein.

Methods for preparing the conjugates of the invention have been described e.g. in WO01/61017. For instance, TNF can be fused with the CNGRCG (SEQ ID NO:1) or SCNGRCG (SEQ ID NO:6) peptide by genetic engineering or by chemical synthesis.

### EXAMPLES

### Materials and methods

**Production of murine and human NGR-TNF in E.coli cells.** cDNAs coding for murine and human CNGRCG-TNF (NGR-TNF) were produced by recombinant DNA technology and cloned in the pET-11 plasmid (Novagen, Madison, WI) as previously described (Curnis et al. 2000). cDNA expression was obtained in BL21(DE3) Escherichia coli cells (Novagen) according to the pET11 manufacture's instruction. The products were purified from bacterial lysates by ammonium sulfate precipitation, hydrophobic interaction chromatography on Phenyl-Sepharose 6 Fast Flow (Pharmacia- Upjohn), ion exchange chromatography on DEAE-Sepharose Fast Flow (Pharmacia-Upjohn), gel filtration chromatography on Sephacryl-S-300 HR (Pharmacia-Upjohn). All solutions used in the chromatographic steps were prepared with sterile and endotoxin-free water (Salf, Bergamo, Italy). **Production of murine and human S-NGR-TNF in E.coli cells.** Murine SCNGRCG-TNF fusion protein (called S-NGR-TNF) was expressed in *E.coli* cells (BL21 Star (DE3)) using the pET101D plasmid (Invitrogen). Human S-NGR-TNF fusion protein was expressed in *E.coli* cells (BL21 Star (DE3)) using the pET101D or the pET11 plasmids.

Murine S-NGR-TNF was purified form cell extracts, obtained by cell sonication and centrifugation, by affinity chromatography on sTNF-R1 (etanercept)-agarose column. The product, which was eluted from the column with a denaturing buffer containing 7 M urea in 100 mM Tris-HCl, pH 7.3, was refolded by dialysis against 333 volumes of 2.33 M urea, 100 mM Tris-HCl, pH 7.3 (1 h, at 4°C), followed by 0.77 M urea, 100 mM Tris-HCl, pH 7.3 (1 h, at 4°C), and 0.26 M urea, 100 mM Tris-HCl, pH 7.3 (1 h, at 4°C). Finally the product were dialyzed against 333 volumes of 100 mM Tris-HCl (16 h, at 4°C), centrifuged, filtered through a 0.22 µm membrane (Nalgene, Rochester, NY), and gel filtered through an HR Sephacryl S-300 column (180 ml) pre-equilibrated with 0.15 M sodium chloride, 25 mM HEPES, pH 7.3. About 1-2 mg of refolded protein were recovered from 1 liter of cell culture.

Human S-NGR-TNF has been produced in a similar manner. About 0.1 mg of human S-NGR-TNF were recovered from 1 liter of cell culture using the pET101D. The yield was markedly increased when human S-NGR-TNF was expressed using the pET11 plasmid, as in this case >100 mg of S-NGR-TNF were produced with one liter of cell culture.

**Production of human NGR-TNF in *Pichia pastoris* cells.** The cDNA encoding for human NGR-TNF was cloned into the pPIC9K plasmid (Proteogenix). Pichia pastoris cells (strain GS115 and K) were then electroporated with the recombinant plasmid coding for CNGRCG-TNF fused to the C-terminus of the alpha mating factor secretion signal peptide (provided by the pPIC9K expression plasmid) to promote the secretion into the culture medium. Cell cultures were induced at 28°C by adding methanol (1%) for 48 h.

### Murine and human NGR-TNF are heterogeneous mixtures of compounds characterized -17Da, +0Da, +42Da, +58Da and, in the case of murine NGR-TNF, +100Da.

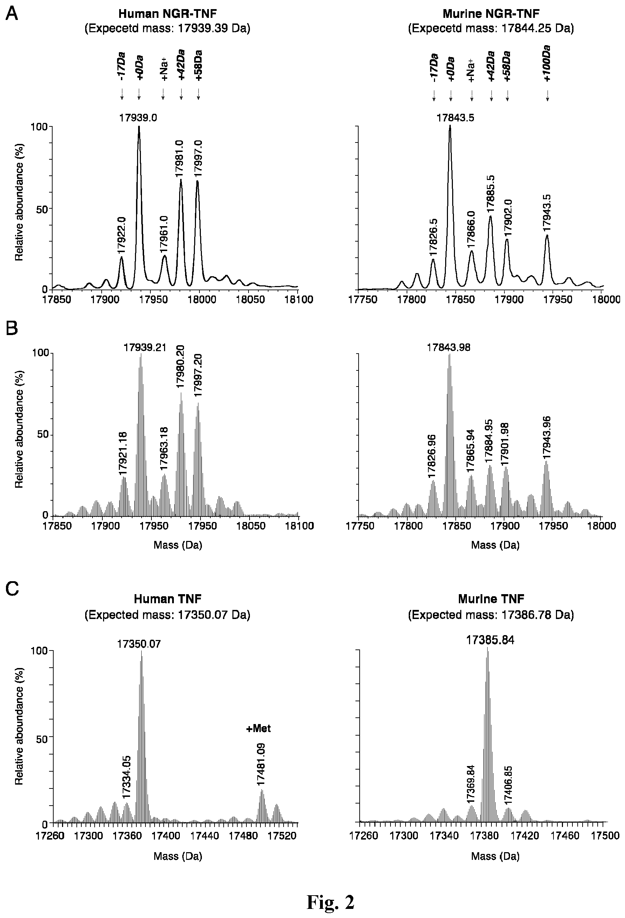

Mass spectrometry (MS) analysis of human NGR-TNF showed that the subunits of this product are a heterogeneous mixture of -17Da, +0Da, +42Da and +58Da forms **(****Fig.2****),** as previously reported for the human NGR-TNF used in clinical studies (Tobias et al. 2013). An additional form of +100KDa was also observed in murine NGR-TNF **(****Fig.2****).** The +22Da and other peaks observed in the mass spectra of both products likely correspond to ion adducts. The +42Da, +58Da and +100Da forms were not observed in murine and human TNF produced using the same expression system (Fig. 2C)

A heterogeneous composition was observed also when murine NGR-TNF was expressed in BL21 Rosetta (DE3) *E.coli* cells or when purified by a different method based on affinity chromatography on a sTNF-R1 (etanercept)-agarose column (not shown).

### The different forms of NGR-TNF are related to modification of the N-terminal sequence

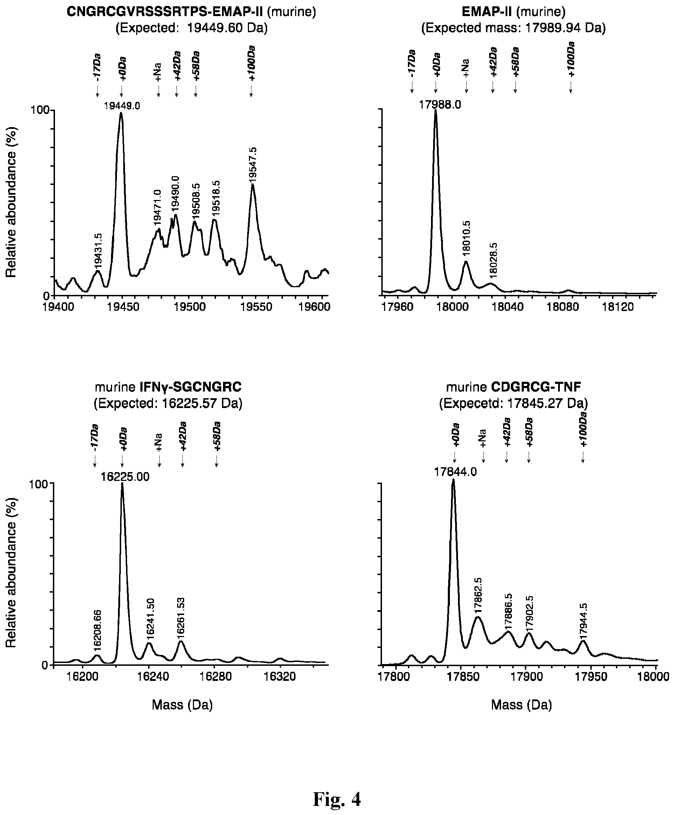

The location of the molecular modifications on NGR-TNF was then investigated. To this aim murine NGR-TNF was a) reduced with ditiothreitol, b) alkylated with iodoacetamide, and c) digested with Asp-N, a protease that can cleave the protein at the N-side of aspartate residues. MS analysis of the product showed that the N-terminal fragment CNGRCGLRSSSQNSS (SEQ ID NO: 37) was heterogeneous, showing again the +0, +42, +58 and + 100 Da forms **(****Fig. 3****).** These data suggest that the chemical modifications of NGR-TNF were located on its N-terminal region (corresponding to CNGRCGVRSSSRTPS (SEQ ID NO:18) in human NGR-TNF). According to this view, no post-translational modifications and no heterogeneity was observed when inventors expressed human or murine TNF in *E.coli* cells **(Table 1).** Furthermore, when inventors replaced the TNF domain of human NGR-TNF with murine EMAP-II, a different cytokine, and expressed the CNGRCGVRSSSRTPS-EMAP-II conjugate in *E.coli* cells inventors still observed molecular heterogeneity, but not when inventors expressed EMAP-II alone **(****Fig. 4** **and Table 1).** Thus, the N-terminal sequence was responsible for the molecular heterogeneity of both NGR-TNF and NGR-EMAP.

### The different forms of NGR-TNF are related to modification of the N-terminal CNGRC sequence

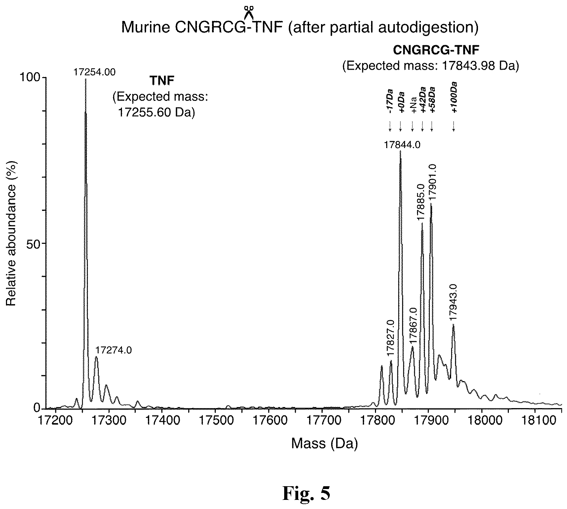

To finely map the location of the molecular modifications of NGR-TNF inventors then replaced the CNGRCG (SEQ ID NO: 1) sequence of murine NGR-TNF with ACDCRGDCFCG (SEQ ID NO:19) and generated the ACDCRGDCFCG-TNF conjugate. No molecular heterogeneity was observed in this case **(Table 1),** suggesting that only the CNGRCG (SEQ ID NO:1) sequence of the N-terminal region was crucial for the modifications. According to this view, removal of CNGRCG (SEQ ID NO:1) from NGR-TNF by partial autoproteolysis for 6 h at 37 °C in ammonium bicarbonate buffer, pH 8.5, resulted in a homogeneous product with a mass corresponding to that of unmodified TNF subunits (17254 Da) **(****Fig. 5****).** These data indicate that the chemical modifications of NGR-TNF were located within the CNGRCG (SEQ ID NO:1) targeting domain.

### Modification of CNGRCG (SEQ ID NO:1) occurs only when this motif is fused with protein N-terminus, but not when is embedded in the protein sequence or fused with protein C-terminus

To assess whether the CNGRCG (SEQ ID NO:1) modification in *E. coli* cells depends on its position in the fusion protein inventors analysed, by mass spectrometry, other NGR-cytokine fusion proteins produced by recombinant DNA technology in *E.coli* cells. No molecular heterogeneity was observed when the SGCNGRC (SEQ ID NO:20) sequence was fused to the C-terminus of IFNgamma **(****Fig. 4** **and Table 1).** Furthermore, no molecular heterogeneity was observed with OmpT-NGR-TNF, a fusion protein with the CNGRCG (SEQ ID NO:1) sequence inserted between the OmpT leader sequence (for periplasmic expression) and TNF. Although inclusion bodies and no periplasmic expression was observed in *E.coli* BL21 Star (DE3), the product was homogeneous **(****Fig. 6** **and Table 1).** It appears, therefore, that molecular heterogenity of the CNGRCG (SEQ ID NO:1) domain occurs only when this domain is fused to the N-terminus of proteins.

### The N-terminal cysteine residue NGR-TNF is partially acetylated and account for the +42Da form

Considering that the weight of an acetyl group is 42 Da, inventors hypothesized that the +42Da forms of human and murine NGR-TNF correspond to molecules with an N-terminal acetyl-cysteine residue. According to this view NGRAHA-TNF, a conjugate lacking the N-terminal cysteine and expressed in *E.coli* cells, was homogeneous and devoid of +42Da forms **(Table 1).** To further assess this hypothesis, inventors exploited an antibody capable of recognizing acetyl-CisoDGRCGVRY (SEQ ID NO: 17), but not CisoDGRCGVRY (SEQ ID NO: 17) peptides **(****Fig. 7A****),** and analyzed its binding to murine or human NGR-TNF **(****Fig. 7B****).** As expected, the antibody could recognize both products, strongly suggesting that a) the +42Da forms corresponded to molecules with an N-terminal acetyl-cysteine, and b) both products were partially deamidated. Possibly, the +58Da modification represents an oxidized form (+16Da) of the +42Da form. Further studies are necessary to clarify this point.

To assess the importance of the second aminoacid residue for protein modification inventors then prepared and analysed the CDGRCG (SEQ ID NO:38)-TNF conjugate. Interestingly, this product contained little or no modified forms **(****Fig. 4** and **Table 1),** suggesting that the presence of a free cysteine at the N-terminus is not sufficient *di per se* for protein modification, but that the concomitant presence of cysteine followed by an asparagine residue (CN) is necessary.

### EXAMPLE 1

### The murine SCNGRCG-TNF (S-NGR-TNF) produced by recombinant DNA technology, is a homogeneous product lacking the +42Da, +58Da and +100Da forms.

Given the crucial role of the N-terminal CN residues of NGR-TNF for protein modification during production and storage, inventors hypothesized that changing this sequence by adding an extra-residue might reduce the molecular heterogeneity. To verify this hypothesis, inventors expressed the murine SCNGRCG-TNF fusion protein (called S-NGR-TNF) in *E.coli* cells (BL21 Star (DE3)) using the pET101D plasmid (Invitrogen) for the fusion protein. SDS-PAGE analysis of murine S-NGR-TNF and NGR-TNF under reducing and non-reducing conditions showed bands corresponding to the ∼17 kDa subunits, in both cases **(****Fig. 8A****).** However, S-NGR-TNF, like TNF, was more homogeneous than NGR-TNF by mass-spectrometry analysis **(****Fig. 8B****).** These data strongly support the hypothesis blocking the alpha-aminogroup of the cysteine-1 of NGR-TNF with a serine residue prevents all CNGRCG (SEQ ID NO:1) modifications during production and storage. This may also explain the observation that the OmpT-NGR-TNF fusion protein, produced as inclusion bodies in *E.coli* cells, is homogeneous **(Table 1).**

### Human S-NGR-TNF produced by recombinant DNA technology, is a homogeneous product lacking the +42Da, +58Da and +100Da forms.

Inventors then investigated whether also human S-NGR-TNF is homogeneous upon expression in in *E.coli* cells. The protein was expressed in *E.coli* cells (BL21 Star (DE3) using the pET101D plasmid and purified form cell extracts by affinity chromatography on sTNF-R1 (etanercept)-agarose column. About 0.1 mg of human S-NGR-TNF were recovered from 1 liter of cell culture using the pET101D. The yield was markedly increased when human S-NGR-TNF was expressed using the pET11 plasmid **(****Fig. 9A****),** as in this case >100 mg of S-NGR-TNF were produced with one liter of cell culture.

SDS-PAGE analysis of affinity purified products (obtained with both low and high expression vectors) showed bands corresponding to human S-NGR-TNF subunits in both cases **(****Fig. 9A****).** Both products were more homogeneous than human NGR-TNF by mass spectrometry analysis **(****Fig. 9B****).** Thus, also in the case of human S-NGR-TNF the addition of an N-terminal serine residue prevents CNGRCG (SEQ ID NO:1) modifications in *E.coli* cells, either when expressed at low or high levels.

### The in vitro cytotoxic activity S-NGR-TNF is similar to that of NGR-TNF

To assess whether the addition of a serine residue to NGR-TNF affects its capability to recognize the TNF receptors and, consequently, its biological activity, inventors then analyzed the cytotoxic activity of TNF, NGR-TNF and S-NGR-TNF in the LM-cells cytolytic assay. The results show that the EC₅₀ of TNF, NGR-TNF and S-NGR-TNF are very similar **(****Fig. 8C****),** suggesting that neither the CNGRCG (SEQ ID NO:1) nor the SCNGRCG (SEQ ID NO:6) domains impair TNF receptor recognition on these cells. Accordingly, the in vitro binding of S-NGR-TNF and NGR-TNF to sTNF-R1 (etanercept) was similar to that of TNF **(****Fig. 8D****).**

### The SCNGRCG (SEQ ID NO:6) sequence can interact with CD13 with an affinity greater than that of CNGRCG (SEQ ID NO:1) or acetyl-CNGRCG

To rule out the possibility that the addition of the serine residue to the N-terminus of NGR-TNF abrogates or reduces its affinity for CD13 inventors performed enzyme kinetics inhibition assays with CD13 (aminopeptidase N) and SCNGRCGVRY (SEQ ID NO: 3), CNGRCGVRY (SEQ ID NO:4) or acetyl-CNGRCGVRY (corresponding to the N-terminal region of human S-NGR-TNF and NGR-TNF). The results showed that the Ki value of SCNGRCGVRY (SEQ ID NO: 3) for CD13 were 3-5 fold lower than that of CNGRCGVRY (SEQ ID NO:4) in different experimental conditions **(****Fig. 10** **and Table 2),** pointing to a higher affinity. Thus, the addition of the serine residue to NGR-TNF does not impair its capability to recognize CD13.

### The SCNGRCG (SEQ ID NO:6) sequence has a lower propensity to undergo deamidation than CNGRCG (SEQ ID NO:1)

To assess whether the addition of the serine residue to CNGRCG (SEQ ID NO:1) might affect its deamidation propensity inventors synthetized the SCNGRCGVRY (SEQ ID NO: 3) and CNGRCGVRY (SEQ ID NO:4) peptides and studied their stability upon incubation at 37°C in PBS, pH 7.4, by mass spectrometry. Notably, after 2 h of incubation, a -17Da form (corresponding the succinimide intermediate of the deamidation reaction) was observed with CNGRCGVRY (SEQ ID NO:4), but not with SCNGRCGVRY (SEQ ID NO: 3) **(****Fig. 11****).** This suggests that the addition of a serine residue has reduced the propensity of the NGR motif to undergo deamidation. Of note, although the +1Da deamidation product (DGR/isoDGR) was observed after 32 h of incubation in both cases, a large amount of the -17Da form was still present in CNGRCGVRY (SEQ ID NO:4), but not in SCNGRCGVRY (SEQ ID NO: 3) **(****Fig. 11****).** These data indicate that the succinimide-derivative of CNGRCG (SEQ ID NO:1), but not that of SCNGRCGVRY (SEQ ID NO: 3), is relatively stable to hydrolysis, strongly suggesting that the -17Da form observed in NGR-TNF correspond to the succinimide intermediate.

A similar experiment was then performed with peptides in 0.1 M ammonium bicarbonate, pH 8.5, a condition known to force Asn deamidation (Curnis et al. 2006). In this buffer both products were rapidly converted to the corresponding Asp/isoAsp forms, indicating that in this condition the -17Da succinimide intermediate is rapidly hydrolyzed also in the case of CNGRCGVRY (SEQ ID NO:4).

Overall, these data suggest that the addition of a Ser residue to the CNGRCG (SEQ ID NO:1) targeting domain of NGR-TNF increases its stability and reduces the formation of the -17Da succinimide-derivative in physiological buffers. Furthermore, the succinimide-derivative of S-NGR-TNF, when formed, is more prone to hydrolysis.

### The deamidation products of S-NGR-TNF bind integrins with an affinity similar to that of the deamidation products of NGR-TNF

To assess whether the isoDGR derivative of S-NGR-TNF and NGR-TNF targeting domains can recognize integrins with similar affinities inventors then analysed the binding of SCisoDGRCGVRY (SEQ ID NO: 17), CisoDGRCGVRY and acetyl-CisoDGRCGVRY to αvβ3, αvβ5, αvβ6 αvβ8 and α5β1 integrins using a competitive binding assay previously described (Curnis et al. 2010). The results showed that SCisoDGRCGVRY could bind these integrins with Ki values similar to those of CisoDGRCGVRY and acetyl-CisoDGRCGVRY **(Table 3).** As expected SCNGRCGVRY (SEQ ID NO: 3) and SCDGRCGVRY (SEQ ID NO:22), tested in parallel, showed no or very low affinity for all integrins. Considering that human NGR-TNF contains acetylated and non-acetylated forms and that both CNGRC (SEQ ID NO:5) and acetyl-CNGRC can undergo deamidation (Curnis et al. 2010), inventors can conclude that the isoDGR derivative of S-NGR-TNF, if formed, behave in a manner similar to those of the isoDGR derivatives of NGR-TNF in terms of integrin recognition.

### S-NGR-TNF is less prone than NGR-TNF to generate integrin binding site upon incubation

To further assess the hypothesis that S-NGR-TNF is more stable and less prone to generate isoDGR-integrin binding sites than NGR-TNF inventors performed EA.hy926 endothelial cell adhesion assays using microtiterplates coated with these proteins. As expected, cell adhesion was observed to plates coated with NGR-TNF, but not to plates coated with S-NGR-TNF **(****Fig. 12****).** When treated the protein-coated microtiterplates with 0.1 M ammonium bicarbonate buffer, pH 8.5, to force deamidation, inventors observed cell adhesion to S-NGR-TNF, indicating that this protein was present. However, a longer time of incubation under deamidation conditions was necessary for S-NGR-TNF, compared to NGR-TNF, to obtain cell adhesion **(****Fig. 12****).** This behavior likely reflects a further improvement in the CNGRCG (SEQ ID NO: 1) stability when fused to TNF

### The addition of a serine residue to the N-terminus of NGR-TNF does not increase its toxicity does not reduce its anti-tumor activity when injected at high doses in tumor mouse models

To assess the impact of the additional serine residue added to the N-terminus of NGR-TNF on its toxicity and anti-tumor activity we injected high-dose NGR-TNF or S-NGR-TNF to mice bearing subcutaneous RMA-T lymphomas (n=6) and evaluated the loss of animal weight. Administration of 2 µg of NGR-TNF at day 11 after tumor implantation caused a marked loss of animal weight (0.89 ± 0.29 g), indicating that this treatment was toxic. Three days later the mice regained the original weight. Subsequent administration of 6 µg at day 18 caused a loss of 1.28 ± 0.29 g, indicating that this dose, as expected, was even more toxic **(****Fig. 13A****, upper panel).** Similar loss of body weight was observed with S-NGR-TNF) **(****Fig. 13A****, upper panel),** suggesting that the modification of the N-terminus of NGR-TNF with a serine residue did not affect its toxicity. Of note, slightly stronger antitumor effects were observed with S-NGR-TNF, compared to NGR-TNF.

### The addition of a serine residue to the N-terminus of NGR-TNF does not reduce its anti-tumor activity when injected at low doses in tumor mouse models

To assess whether the addition of the Ser residue to NGR-TNF might affect its anti-tumor activity at low dose, inventors then analyzed the therapeutic activity of NGR-TNF and S-NGR-TNF (25 or 50 pg, i.p.) using the subcutaneous WEHI-164 fibrosarcoma murine model. Both conjugates delayed tumor growth to similar extent **(****Fig. 14****),** indicating that the addition of the S residue to NGR-TNF does not impair its anti-tumor activity. No evidence of toxicity was obtained in both cases. Of note a slightly stronger anti-tumor effects were obtained with 25 pg of S-NGR-TNF compared to 25 pg of NGR-TNF.

Finally, to assess whether low-dose S-NGR-TNF can exert synergistic effects with chemotherapy, as previously observed for NGR-TNF, inventors performed *in vivo* experiments with S-NGR-TNF and NGR-TNF in combination with melphalan, a chemotherapeutic drug, using the murine RMA lymphoma model **(****Fig. 15****).** Both drugs could enhance the efficacy of melphalan in this model, indicating that S-NGR-TNF both NGR-TNF can synergize with chemotherapy. Again a slightly stronger antitumor effect was observed with S-NGR-TNF compared to NGR-TNF.

### S-NGR-TNF does not exacerbate the toxicity of melphalan.

To verify that S-NGR-TNF does not exacerbate the toxicity of melphalan, we then analysed the loss of body weight in response to the combined treatment with low-dose S-NGR-TNF and melphalan. We found that the combination of S-NGR-TNF (100 pg) with melphalan (50 µg) did not increased the loss of animal weight compared to controls **(****Fig. 16****, upper panel),** despite significant increase of antitumor activity was observed **(****Fig. 16****, lower panel)**

These data suggest that addition of a serine residue to the N-terminus of NGR-TNF does not increase, on the one hand the inherent toxicity of NGR-TNF, and on the other hand it does not contribute to exacerbate the toxicity of melphalan.

To verify that S-NGR-TNF does not exacerbate the toxicity of melphalan, we then analysed the loss of body weight in response to the combined treatment with low-dose S-NGR-TNF and melphalan. We found that the combination of S-NGR-TNF (100 pg) with melphalan (50 µg) did not increased the loss of animal weight compared to controls **(****Fig. 16B****, upper panel),** despite significant increase of antitumor activity was observed **(****Fig. 16B****, lower panel)**

These data suggest that addition of a serine residue to the N-terminus of NGR-TNF does not increase, on the one hand the inherent toxicity of NGR-TNF, and on the other hand it does not contribute to exacerbate the toxicity of melphalan.

### Conclusion

The results of biochemical studies show that the serine residue added to the N-terminus of NGR-TNF abrogates the formation of the -17Da, +42Da, +58 Da and +100Da forms (upon expression in *E.coli* cells, purification and storage) and improves its stability to deamidation. Furthermore, the results of *in vitro* and *in vivo* biological studies indicate that the addition of the serine residue to NGR-TNF does not impair its capability to recognize CD13 and TNF receptor, does not reduce its therapeutic activity, and does not increase its toxicity. In contrast, the addition of the serine the addition of the serine residue to NGR-TNF improves its affinity for CD13 and its anti-tumor activity.

### EXAMPLE 2

To assess whether the problem of molecular heterogeneity of NGR-TNF can be solved by fusing CNGRCG (SEQ ID NO:1) with the C-terminus of a polypeptide sequences that is cleaved upon expression in an appropriate host, inventors then produced human NGR-TNF in *Pichia pastoris* cells, engineered to secrete human CNGRCG-TNF. In this system the cDNA coding for CNGRCG-TNF is fused to the C-terminus of the alpha-mating factor secretion-signal peptide **(****Figure 17****)** to promote the secretion into the culture medium through the addition of methanol. Thus, using this system the protein is produced with the leader sequence that is cleaved upon secretion. SDS-PAGE analysis of the cell supernatant obtained with various clones showed that NGR-TNF can be secreted at high levels **(****Figure 18B****).** Mass spectrometry analysis of the affinity purified product showed that NGR-TNF produced in this manner was homogeneous, characterized by the expected molecular weight, and devoid of the +42, +58Da **(****Figure 18C****).**

The references cited throughout this application are incorporated for all purposes apparent herein and in the references themselves as if each reference was fully set forth. For the sake of presentation, specific ones of these references are cited at particular locations herein. A citation of a reference at a particular location indicates a manner(s) in which the teachings of the reference are incorporated. However, a citation of a reference at a particular location does not limit the manner in which all of the teachings of the cited reference are incorporated for all purposes.

It is understood, therefore, that this invention is not limited to the particular embodiments disclosed, but is intended to cover all modifications which are within the spirit and scope of the invention as defined by the appended claims; the above description; and/or shown in the attached drawings.

### Tables

**Table 1. Characterization of recombinant proteins expressed in E.Coli cells, by mass spectrometry analysis**

| Protein^{a} | Expression | | Found molecular forms (Da)^{b} | | |
|---|---|---|---|---|---|
| | Plasmid | *E.Coli* strain | | | |
| | | | +*42* | +*58* | +*100* |
| Human TNF | pET11 | BL21(DE3) | - | - | - |
| Human CNGRCG-TNF | pET11 | BL21(DE3) | + | + | + |
| Murine TNF | pET11 | BL21(DE3) | - | - | - |
| Murine CNGRCG-TNF | pET11 | BL21(DE3) | + | + | + |
| Murine CDGRCG-TNF | pET101/D | BL21(DE3) | +/- | +/- | +/- |
| Murine OmpT-CNGRCG-TNF | pET12 | BL21 Star (DE3) | -^{c} | -^{c} | -^{c} |
| Murine ACDCRGDCFCG-TNF | pET11 | BL21(DE3) | - | - | - |
| Murine NGRAHA-TNF | pET101/D | BL21 Star (DE3) | - | - | - |
| Murine EMAP-II^{d} | pET101/D | BL21(DE3) | - | - | - |
| Murine CNGRCGVRSSSRTPS-EMAP-II^{d} | pET101/D | BL21(DE3) | + | + | + |
| Human His_{tag}-vasostatin-1^{e} | pET100/D | BL21 Star (DE3) | - | - | - |
| Human His_{tag}-CNGRCG-vasostatin-1^{e} | pET101/D | BL21(DE3) | - | - | - |
| Murine IFNγ-SGCNGRC^{f} | pET11 | BL21(DE3) | - | - | - |

| | | | | | |
|---|---|---|---|---|---|
| a) *Bold,* aminoacid sequence (single letter code) added by recombinant UNA technology to the following proteins: *TNF,* Tumor Necrosis Factor-α; *EMAP, Endothelial-Monocyte Activating Polypeptide-II; IFN-*γ; *Interferon-*γ. OmpT, outer membrane protein T leader sequence; His_{tag}-Xpress, six-histidine tag and Xpress fusion product. b) by ESI-MS c) As determined by the analysis of the LLGIVLTTPIAISSFASTCNGR (SEQ ID NO: 21) fragment obtained by trypsin digestion (see **Fig. 6**). d) Produced as described in (Crippa et al. 2008). e) His_{tag}: six-histidine tag-Xpress epitope fused to vasostatin-1. f) Produced as described in (Curnis et al. 2005). | | | | | |

**Table 2. CD13 inhibitory activity of SCNGRCGVRY (SEQ ID NO: 3), CNGRCGVRY (SEQ ID NO:4), and acetyl-CNGRCGVRY peptides**

| *Peptide* | *CD13 inhibitory activity (µM)* | | | |
|---|---|---|---|---|
| | *Tris-HCl buffer ^{a}* | | *Potassium-phosphate buffer ^{b}* | |
| | *n ^{c}* | *Ki ^{d}* | *n* | *Ki* |
| CNGRCGVRY (SEQ ID NO:4) | 4 | 35.6 ± 4.0 | 1 | 76.5 |
| acetyl-CNGRCGVRY | 3 | 38.1 ± 9.8 | 1 | 50.7 |
| SCNGRCGVRY (SEQ ID NO:3) | 4 | 6.8 ± 0.8 | 2 | 25.0 ± 0.5 |

| | | | | |
|---|---|---|---|---|
| a) 50 mM Tris-HCl buffer, pH 7.4. b) 60 mM potassium phosphate buffer, pH 7.4 c) *n*, number of independent experiments, each in duplicates. d) *Ki,* inhibitory constant was calculated using Prism software (mean ± SE). Each experiment was performed with 4 technical replicates | | | | |

**Table 3. Integrin binding affinity of the deamidation products of SCNGRCGVRY (SEQ ID NO: 3) and CNGRCGVRY (SEQ ID NO:4) peptides, as determined by competitive binding assay and expressed as inhibition constants (Ki, nM)^{a}**

| *Peptide* | *(Ki)* (mean± SE) | | | | | | | | | |
|---|---|---|---|---|---|---|---|---|---|---|
| | αvβ3 | | αvβ5 | | αvβ6 | | αvβ8 | | α5β1 | |
| | *n* ^{c} | *Ki* | *n* | *Ki* | *n* | *Ki* | *n* | *Ki* | *n* | *Ki* |
| SCNGRCGVRY (SEQ ID NO: 3) | *2* | >10000 | *2* | >10000 | *2* | >10000 | *2* | >10000 | *2* | >10000 |
| SCisoDGRCGVRY | *4* | 17 ± 5 | *5* | 77 ± 64 | *4* | 25 ± 9 | *4* | 156 ± 77 | *3* | 11 ± 1 |
| SCDGRCGVRY (SEQ ID NO:22) | *2* | 3342 ± 932 | *2* | 779 ± 241 | *2* | 5497 ± 2440 | *2* | 21350 ± 6140 | *2* | 4793 ± 207 |
| CisoDGRCGVRY | *9* | 11 ± 2 | *10* | 391 ± 119 | *11* | 131 ± 39 | *7* | 554 ± 97 | *6* | 75 ± 25 |
| ac-CisoDGRCGVRY | *8* | 3 ± 1 | *9* | 21 ± 5 | 7 | 5 ± 1 | *6* | 18 ± 5 | *4* | 4 ± 1 |

| | | | | | | | | | | |
|---|---|---|---|---|---|---|---|---|---|---|
| a) Competitive binding of acetyl-CisoDGRCG/streptavidin-peroxidase to integrins. The binding assay was performed using integrin-coated microtiterplates as previously described (Curnis et al. 2013). b) *n*, number of independent experiments, each in duplicate. | | | | | | | | | | |

### References

Arap, W., R. Pasqualini, and E. Ruoslahti. 1998. 'Cancer treatment by targeted drug delivery to tumor vasculature in a mouse model', Science, 279: 377-80.
Bhagwat, S. V., J. Lahdenranta, R. Giordano, W. Arap, R. Pasqualini, and L. H. Shapiro. 2001. 'CD13/APN is activated by angiogenic signals and is essential for capillary tube formation', Blood, 97: 652-59.
Buehler, A., M. A. van Zandvoort, B. J. Stelt, T. M. Hackeng, B. H. Schrans-Stassen, A. Bennaghmouch, L. Hofstra, J. P. Cleutjens, A. Duijvestijn, M. B. Smeets, D. P. de Kleijn, M. J. Post, and E. D. de Muinck. 2006. 'cNGR: a novel homing sequence for CD13/APN targeted molecular imaging of murine cardiac angiogenesis in vivo', Arterioscler Thromb Vasc Biol, 26: 2681-7.
Calcinotto, A., M. Grioni, E. Jachetti, F. Curnis, A. Mondino, G. Parmiani, A. Corti, and M. Bellone. 2012. 'Targeting TNF-alpha to Neoangiogenic Vessels Enhances Lymphocyte Infiltration in Tumors and Increases the Therapeutic Potential of Immunotherapy', Journal of Immunology*.*
Corti, A., and F. Curnis. 2011. 'Tumor vasculature targeting through NGR peptide-based drug delivery systems', Current pharmaceutical biotechnology, 12: 1128-34.
Corti, A., F. Curnis, W. Arap, and R. Pasqualini. 2008. 'The neovasculature homing motif NGR: more than meets the eye', Blood, 112: 2628-35.
Corti, A., F. Curnis, G. Rossoni, F. Marcucci, and V. Gregorc. 2013. 'Peptide-mediated targeting of cytokines to tumor vasculature: the NGR-hTNF example', BioDrugs, 27: 591-603.
Crippa, L., A. Gasparri, A. Sacchi, E. Ferrero, F. Curnis, and A. Corti. 2008. 'Synergistic damage of tumor vessels with ultra low-dose endothelial-monocyte activating polypeptide-II and neovasculature-targeted tumor necrosis factor-alpha', Cancer Res, 68: 1154-61.
Curnis, F., G. Arrigoni, A. Sacchi, L. Fischetti, W. Arap, R. Pasqualini, and A. Corti. 2002. 'Differential binding of drugs containing the NGR motif to CD13 isoforms in tumor vessels, epithelia, and myeloid cells', Cancer Research, 62: 867-74.
Curnis, F., A. Cattaneo, R. Longhi, A. Sacchi, A. M. Gasparri, F. Pastorino, P. Di Matteo, C. Traversari, A. Bachi, M. Ponzoni, G. P. Rizzardi, and A. Corti. 2010. 'Critical role of flanking residues in NGR-to-isoDGR transition and CD13/Integrin receptor switching', J Biol Chem, 285: 9114-23.
Curnis, F., A. Gasparri, A. Sacchi, A. Cattaneo, F. Magni, and A. Corti. 2005. 'Targeted delivery of IFN-gamma to tumor vessels uncouples anti-tumor from counter-regulatory mechanisms', Cancer Research, 65: 2906-13.
Curnis, F., R. Longhi, L. Crippa, A. Cattaneo, E. Dondossola, A. Bachi, and A. Corti. 2006. 'Spontaneous formation of L-isoaspartate and gain of function in fibronectin', J Biol Chem, 281: 36466-76.
Curnis, F., A. Sacchi, L. Borgna, F. Magni, A. Gasparri, and A. Corti. 2000. 'Enhancement of tumor necrosis factor alpha antitumor immunotherapeutic properties by targeted delivery to aminopeptidase N (CD13)', Nat. Biotechnol., 18: 1185-90.
Curnis, F., A. Sacchi, and A. Corti. 2002. 'Improving chemotherapeutic drug penetration in tumors by vascular targeting and barrier alteration', Journal of Clinical Investigation, 110: 475-82.
Curnis, F., A. Sacchi, R. Longhi, B. Colombo, A. Gasparri, and A. Corti. 2013. 'IsoDGR-tagged albumin: a new avb3 selective carrier for nanodrug delivery to tumors', Small, 9: 673-78.
Di Matteo, P., G. L. Arrigoni, L. Alberici, A. Corti, C. Gallo-Stampino, C. Traversari, C. Doglioni, and G. P. Rizzardi. 2010. 'Enhanced Expression of CD13 in Vessels of Inflammatory and Neoplastic Tissues', The journal of histochemistry and cytochemistry : official journal of the Histochemistry Society*.*
Dixon, J., L. Kaklamanis, H. Turley, I. D. Hickson, R. D. Leek, A. L. Harris, and K. C. Gatter. 1994. 'Expression of aminopeptidase-n (CD 13) in normal tissues and malignant neoplasms of epithelial and lymphoid origin', Journal of Clinical Pathology, 47: 43-7.
Ferreri, A. J. M., T. Calimeri, G. M. Conte, D. Cattaneo, F. Fallanca, M. Ponzoni, E. Scarano, F. Curnis, A. Nonis, P. Lopedote, G. Citterio, L. Politi, M. Foppoli, S. Girlanda, M. Sassone, S. Perrone, C. Cecchetti, F. Ciceri, C. Bordignon, A. Corti, and N. Anzalone. 2019. 'R-CHOP preceded by blood-brain barrier permeabilization with engineered tumor necrosis factor-alpha in primary CNS lymphoma', Blood, 134:252-262.
Gregorc, V., F. G. De Braud, T. M. De Pas, R. Scalamogna, G. Citterio, A. Milani, S. Boselli, C. Catania, G. Donadoni, G. Rossoni, D. Ghio, G. Spitaleri, C. Ammannati, S. Colombi, F. Caligaris-Cappio, A. Lambiase, and C. Bordignon. 2011. 'Phase I study of NGR-hTNF, a selective vascular targeting agent, in combination with cisplatin in refractory solid tumors', Clinical cancer research : an official journal of the American Association for Cancer Research, 17: 1964-72.
Gregorc, V., R. M. Gaafar, A. Favaretto, F. Grossi, J. Jassem, A. Polychronis, P. Bidoli, M. Tiseo, R. Shah, P. Taylor, S. Novello, A. Muzio, A. Bearz, L. Greillier, F. Fontana, G. Salini, A. Lambiase, and M. O'Brien. 2018. 'NGR-hTNF in combination with best investigator choice in previously treated malignant pleural mesothelioma (NGR015): a randomised, double-blind, placebo-controlled phase 3 trial', Lancet Oncol, 19: 799-811.
Gregorc, V., P. A. Zucali, A. Santoro, G. L. Ceresoli, G. Citterio, T. M. De Pas, N. Zilembo, F. De Vincenzo, M. Simonelli, G. Rossoni, A. Spreafico, M. Grazia Vigano, F. Fontana, F. G. De Braud, E. Bajetta, F. Caligaris-Cappio, P. Bruzzi, A. Lambiase, and C. Bordignon. 2010. 'Phase II study of asparagine-glycine-arginine-human tumor necrosis factor alpha, a selective vascular targeting agent, in previously treated patients with malignant pleural mesothelioma', J Clin Oncol, 28: 2604-11.
Lahdenranta, J., R. L. Sidman, R. Pasqualini, and W. Arap. 2007. 'Treatment of hypoxia-induced retinopathy with targeted proapoptotic peptidomimetic in a mouse model of disease', FASEB J, 21: 3272-8.
Lorusso, D., G. Scambia, G. Amadio, A. di Legge, A. Pietragalla, R. De Vincenzo, V. Masciullo, M. Di Stefano, G. Mangili, G. Citterio, M. Mantori, A. Lambiase, and C. Bordignon. 2012. 'Phase II study of NGR-hTNF in combination with doxorubicin in relapsed ovarian cancer patients', British Journal of Cancer, 107: 37-42.
Luan, Y., and W. Xu. 2007. 'The structure and main functions of aminopeptidase N', Curr Med Chem, 14: 639-47.
Mina-Osorio, P. 2008. 'The moonlighting enzyme CD13: old and new functions to target', Trends Mol Med, 14: 361-71.
Oostendorp, M., K. Douma, T. M. Hackeng, A. Dirksen, M. J. Post, M. A. van Zandvoort, and W. H. Backes. 2008. 'Quantitative molecular magnetic resonance imaging of tumor angiogenesis using cNGR-labeled paramagnetic quantum dots', Cancer Res, 68: 7676-83.
Pasqualini, R., E. Koivunen, R. Kain, J. Lahdenranta, M. Sakamoto, A. Stryhn, R. A. Ashmun, L. H. Shapiro, W. Arap, and E. Ruoslahti. 2000. 'Aminopeptidase N is a receptor for tumor-homing peptides and a target for inhibiting angiogenesis', Cancer Research, 60: 722-7.
Sacchi, A., A. Gasparri, C. Gallo-Stampino, S. Toma, F. Curnis, and A. Corti. 2006. 'Synergistic antitumor activity of cisplatin, paclitaxel, and gemcitabine with tumor vasculature-targeted tumor necrosis factor-alpha', Clin Cancer Res, 12: 175-82.
Shipp, M. A., and A. T. Look. 1993. 'Hematopoietic differentiation antigens that are membrane-associated enzymes: cutting is the key', Blood, 82: 1052-70.
Spitaleri, A., S. Mari, F. Curnis, C. Traversari, R. Longhi, C. Bordignon, A. Corti, G. P. Rizzardi, and G. Musco. 2008. 'Structural basis for the interaction of isoDGR with the RGD-binding site of avbeta 3 integrin', J Biol Chem.
Taylor, A. 1993. 'Aminopeptidases: structure and function', FASEB Journal, 7: 290-8.
Tobias, R., C. Dectchou, C.J Anders, S. Rovetta, P. Belloni, G.P. Rizzardi, and J. McEntire. 2013. 'Multiple reaction monitoring mass spectrometry for biopharmaceutical analysis', Pharmaceutical Technology, Analytical & Bioanalytical Testing: 11-18.
Zarovni, N., L. Monaco, and A. Corti. 2004. 'Inhibition of tumor growth by intramuscular injection of cDNA encoding tumor necrosis factor alpha coupled to NGR and RGD tumor-homing peptides', Human Gene Therapy, 15: 373-82.
Zucali, P. A., M. Simonelli, F. De Vincenzo, E. Lorenzi, M. Perrino, M. Bertossi, R. Finotto, S. Naimo, L. Balzarini, C. Bonifacio, I. Timofeeva, G. Rossoni, G. Mazzola, A. Lambiase, C. Bordignon, and A. Santoro. 2013. 'Phase I and pharmacodynamic study of high-dose NGR-hTNF in patients with refractory solid tumours', British Journal of Cancer, 108: 58-63.

## Claims

1. A conjugate comprising:
- a first peptide of sequence CNGRCG (SEQ ID NO:1) linked to the N-terminus of a protein;
- a compound X linked to the N-terminus of said peptide.

2. The conjugate according to claim 1 which is able to recognize CD13.

3. The conjugate according to claim 1 or 2, wherein the protein is a cytokine, preferably a cytokine endowed of anti-tumor activity, more preferably the cytokine is selected from the group consisting of: tumor necrosis factor (TNF), preferably TNF-alpha or TNF-beta, TNF-related apoptosis inducing ligand (TRAIL), endothelial monocyte activating polypeptide II (EMAP-II), IL12, Interferon Gamma (IFNgamma) and Interferon alpha (IFNalpha).

4. The conjugate according to any one of the previous claims, wherein the compound X is a second peptide of 1-200 amino acid residues, preferably of 1, 2 or 3 amino acid residues.

5. The conjugate according to claim 4, wherein the second peptide consists of: a serine residue or any amino acid with a short side chain, preferably glycine or alanine, an amino acid sequence comprising the IEGR (SEQ ID NO:2) sequence or a leader sequence which is removed upon expression of the conjugate in eukaryotic cells and secretion, preferably an OmpT leader sequence or the alpha mating factor secretion signal peptide.

6. The conjugate according to claim 5 wherein the second peptide consists of a serine and the cytokine is TNF, preferably wherein TNF is human TNF-alpha.

7. The conjugate according to any one of the previous claims wherein the conjugate contains a site for chemical or enzymatic cleavage of the bond between the compound X and the peptide (X-C bond), preferably wherein the cleavage of the X-C bond can be achieved with an aminopeptidase or an endoprotease, preferably with aminopeptidase N (CD13) or with a protease, preferably factor Xa.

8. A nucleic acid encoding for the conjugate according to any one of claims 1-7 or a vector, preferably a plasmid or a viral vector, for gene therapy containing said nucleic acid.

9. A nanoparticle comprising the conjugate according to any one of claims 1-7, preferably the conjugate is adsorbed on the surface of gold nanoparticles.

10. A combination product comprising the conjugate according to any one of claims 1-7 or the nucleic acid of claim 8 or the vector of claim 8 or the nanoparticle of claim 10 and at least one antitumor agent, preferably being a chemotherapeutic agent and/or immunomodulator and/or immune cell, preferably the chemotherapeutic agent is doxorubicin, melphalan, gemcitabine, taxol, cisplatin, vincristine, or vinorelbine, preferably the immunomodulator is an anticancer vaccine or an immune check point blocker, such as anti-PD1 or anti-PDL1 or antiCTLA4 antibodies, preferably the immune cell is a lymphocyte or a genetically modified T-lymphocyte, such as CAR-T cells, or TCR redirected T-cells or NK cells, preferably the further antitumor agent comprises an antibody and a chemotherapeutic agent, such as R-CHOP: rituximab, cyclophosphamide, vincristine, doxorubicin, prednisolone.

11. The conjugate according to any one of claims 1-7 or the nucleic acid of claim 8 or the vector of claim 8 or the nanoparticle of claim 9 or the combination product according to claim 10 for medical use, preferably for use the treatment of tumors, preferably solid tumors, more preferably lymphomas, preferably primary diffuse large B-cell lymphoma of the CNS (PCNSL), brain tumors (e.g. glioblastoma and astrocytoma), sarcoma, melanoma oral or skin squamous cell carcinoma, hepatocellular carcinoma, head and neck, gastroesophageal, colorectal, pancreatic, ovarian, lung, cervix, breast cancer, renal, urothelial or metastasis thereof.

12. A pharmaceutical composition comprising an effective amount of a conjugate as claimed in any one of claims 1-7 or the nucleic acid of claim 8 or the vector of claim 8 or the combination product according to claim 10 or the nanoparticle of claim 9 and at least one antitumor agent, together with at least one pharmaceutically acceptable carrier and/or excipient.

13. A method for producing a homogeneous conjugate comprising the sequence CNGRCG (SEQ ID NO:1) linked to the N-terminus of a protein, said method comprising:
- the expression of a conjugate as defined in claim 7 in prokaryotic or eukaryotic cells, preferably *E.coli* cells and *B. subtilis;*
- chemical or enzymatic cleavage of X-C bond, preferably with an aminopeptidase, an endoproteinase or a protease.

14. A method for producing a homogeneous conjugate comprising the sequence CNGRCG (SEQ ID NO:1) linked to the N-terminus of a protein, said method comprising DNA expression in a host unable to acetylate the alpha-amino group or to modify the CN sequence, said host being preferably eukaryotic cells.

15. A method for producing a homogeneous conjugate comprising the sequence CNGRCG (SEQ ID NO:1) linked to the N-terminus of a protein, said method comprising:
- the expression of said conjugate further comprising a leader sequence which is removed upon expression in eukaryotic cells or plant or animals, preferably in *Pichia Pastoris* cells, CHO cells or baculovirus insect cells systems.
- secretion of the conjugate.
